# EUROPEAN PATENT APPLICATION

(11) **EP 4 592 315 A1**
(43) Date of publication of application: **30.07.2025**
(21) Application number: 23867510.2
(22) Date of filing: 19.09.2023
(51) Int. Cl.: C07K 16/28, C07K 19/00, C07K 16/46, C12N 15/13, A61K 39/395, A61P 35/00, G01N 33/68, G01N 33/574

(54) **ANTIBODY AND USE THEREOF IN RESISTING TUMOR**

(30) Priority: 20.09.2022 CN 202211142594
(71) Applicant: Biotheus Inc., Tangjiawan Town, Xiangzhou District Zhuhai, Guangdong 519080 (CN)
(72) Inventor: LUO, Yi, Zhuhai, Guangdong 519080 (CN); CHEN, Liandi, Zhuhai, Guangdong 519080 (CN); HUANG, Weifeng, Zhuhai, Guangdong 519080 (CN); MIAO, Xiaoniu, Zhuhai, Guangdong 519080 (CN); YAN, Yao, Zhuhai, Guangdong 519080 (CN); WANG, Chao, Zhuhai, Guangdong 519080 (CN)
(74) Representative: Ipsilon
(86) International application number: PCT/CN2023/119761
(87) International publication number: WO 2024/061223

(57) **Abstract**

Provided are an antibody and use thereof in resisting a tumor. Provided are a single-domain antibody capable of specifically binding to 4-1BB or an antigen-binding fragment thereof, and a multi-specific antibody. The antibody has improved anti-tumor activity and lower liver toxicity.

## Description

The present application is based on the application with CN application number of 202211142594.0 and application date of September 20, 2022, and claims its priority. The disclosure of the CN application is hereby incorporated into the present application in its entirety.

### Technical Field

The present application relates to the field of biotherapy technology, and specifically to an antibody targeting 4-1BB and HER2 and use thereof in tumor treatment.

### Background Art

Human epidermal growth factor receptor 2 (HER2/ErbB2/Neu) is a member of the ErbB receptor tyrosine kinase family, which is overexpressed in many solid tumors, and can form a homodimer or a heterodimer with other member of the ErbB family members to trigger the phosphorylation of tyrosine residues within the receptor's cytoplasmic domain, thereby activating multiple signaling pathways in the cell, and promoting cancer cell proliferation and tumoiigenesis^{[1]}. As one of the earliest targets used for tumor treatment, therapeutic drugs targeting HER2 include monoclonal antibodies (mAbs, such as trastuzumab and pertuzumab), tyrosine kinase inhibitors (TKIs, such as neratinib and lapatinib), and antibody-drug conjugates (ADCs, such as T-DM1), etc. These drugs have been widely used in the treatment of breast cancer or gastric cancer with HER2 overexpression and/or amplification^{[2]}. Despite these HER2-targeted therapeutic drugs have good clinical benefits, unmet medical needs persist, and new drugs need to be continuously developed to further improve the clinical efficacy in patients with relapsed or metastatic diseases.

The T cell co-stimulatory receptor TNFRSF9 (4-1BB) is a member of the TNF receptor family and is predominantly expressed on activated T cells. When being activated, it can enhance the effector activity and memory response of T cells^{[3,4]}. Urelumab, a monoclonal antibody drug targeting 4-1BB, exerts therapeutic effect by binding and activating 4-1BB on T cells. Unfortunately, significant liver-related toxicity was observed during the clinical development of Urelumab.

The development of anti-tumor drugs with better efficacy and higher safety is still an urgent problem to be solved in this field.

### Contents of the present invention

The present application discloses a single-domain antibody with high binding activity to 4-1BB, and a multi-specific antibody targeting 4-1BB and HER2 developed on this basis. In particular, the present application discloses a HER2xHER2x4-1BB tri-specific antibody, which has unique anti-tumor activity and strong immune memory effect. The antibody is a 1+1 IgG1-like heterodimer antibody composed of trastuzumab and pertuzumab, and its C-terminal is connected to an anti-4-1BB single-domain antibody (sdAb) through a G4S linker. The antibody retains the Fc effector function, which is crucial for trastuzumab and pertuzumab to exert anti-tumor activity. In addition, the antibody has lower liver toxicity and improved safety.

### Single-domain antibody

In one aspect, the present application provides a single-domain antibody or antigen-binding fragment thereof capable of specifically binding to 4-1BB, wherein the single-domain antibody comprises:
(a) a CDR1, which has a sequence as set forth in SEQ ID NO: 43, or a sequence having a substitution, deletion or addition of one or more amino acids (e.g., a substitution, deletion or addition of 1, 2 or 3 amino acids) as compared to the sequence as set forth in SEQ ID NO: 43;
(b) a CDR2, which has a sequence as set forth in SEQ ID NO: 44, or a sequence having a substitution, deletion or addition of one or more amino acids (e.g., a substitution, deletion or addition of 1, 2 or 3 amino acids) as compared to the sequence as set forth in SEQ ID NO: 44; and
(c) a CDR3, which has a sequence as set forth in SEQ ID NO: 45, or a sequence having a substitution, deletion or addition of one or more amino acids (e.g., a substitution, deletion or addition of 1, 2 or 3 amino acids) as compared to the sequence as set forth in SEQ ID NO: 45.

In some embodiments, the single-domain antibody or antigen-binding fragment thereof comprises: a CDR1 as set forth in SEQ ID NO: 43, a CDR2 as set forth in SEQ ID NO: 44, and a CDR3 as set forth in SEQ ID NO: 45.

The single-domain antibody comprises an amino acid sequence selected from the following:
(i) a sequence as set forth in SEQ ID NO: 7;
(ii) a sequence having a substitution, deletion or addition of one or more amino acids (e.g., a substitution, deletion or addition of 1, 2, 3, 4 or 5 amino acids) as compared to the sequence as set forth in SEQ ID NO: 7; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 7.

In some embodiments, the substitution is a conservative substitution.

The term "4-1BB", also known as CD137 or TNFRSF9 (the member 9 of TNF receptor superfamily), is a member of the TNF receptor superfamily (TNFRSF), and is a co-stimulatory molecule that is expressed upon activation of immune cells (both innate immune cells and adaptive immune cells). 4-1BB plays an important role in regulating the activity of various immune cells. As used herein, 4-1BB may be derived from a mammal, such as *Homo sapiens* (human) (NCBI accession number NP_001552.2).

In some embodiments, the single-domain antibody specifically binds to 4-1BB epitope CRD-4.

### Polypeptide construct

The present application also provides a polypeptide construct capable of specifically binding to 4-1BB, which comprises the single-domain antibody or antigen binding fragment thereof as described above, and an immunoglobulin Fc domain.

In some embodiments, the immunoglobulin Fc domain is linked directly or via a peptide linker to the N-terminal and/or C-terminal (e.g., C-terminal) of the single-domain antibody or antigen binding fragment thereof.

In some embodiments, the peptide linker has a structure represented by (GₘXₙ)ₗG_{m'}, wherein m, m', n and l are each independently selected from 0, 1, 2, 3, 4, 5, 6, 7, 8, 9 and 10, and X is selected from A and S; preferably, m is selected from 1, 2, 3, 4 and 5, n is selected from 1 and 2, l is selected from 1, 2, 3, 4, 5, 6, 7, 8, 9 and 10, m' is selected from 0 and 1, and X is selected from A and S.

In some embodiments, the peptide linker has an amino acid sequence as set forth in SEQ ID NO: 17 or 18.

In some embodiments, the immunoglobulin Fc domain is an Fc domain of IgG (e.g., an Fc domain of IgG1, for example, comprising CH2 and CH3).

In some embodiments, the immunoglobulin Fc domain comprises a sequence as set forth in SEQ ID NO: 19, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion or addition of one or more amino acids (e.g., a substitution, deletion or addition of 1, 2, 3, 4 or 5 amino acids) as compared thereto.

In some embodiments, the immunoglobulin Fc domain comprises a sequence as set forth in SEQ ID NO: 19 or 20.

In some embodiments, the polypeptide construct comprises or consists of an amino acid sequence as set forth in SEQ ID NO: 31 or 32.

### Multi-specific antibody

The present application further provides a multi-specific antibody capable of activating 4-1BB signaling only when cross-linked with a tumor cell expressing HER2. In addition, the anti-4-1BB antibody or antigen-binding fragment thereof contained in the multi-specific antibody may be characterized by localization and/or activation only in the tumor microenvironment (TME), and/or significantly reducing liver toxicity compared to existing anti-4-1BB antibodies, while maintaining the efficacy of immune response enhancement and/or tumor treatment.

Specifically, the present application provides a multi-specific antibody, which comprises the single-domain antibody or antigen-binding fragment thereof or the polypeptide construct as described above.

The present application further provides a multi-specific antibody, which comprises a first antigen-binding domain specific for 4-1BB, wherein the first antigen-binding domain comprises the antibody or antigen-binding fragment thereof as described above.

In some embodiments, the first antigen-binding domain comprises the antibody or antigen-binding fragment thereof as described above.

In some embodiments, any of the above multi-specific antibodies specifically binds to 4-1BB, and additionally specifically binds to one or more other targets.

In some embodiments, the target is a tumor antigen.

In some embodiments, the tumor antigen is one or more selected from the following: CD19, CD20, CD22, CD23, CD38, CD40, CD49, CD52, CD56, CD74, CD80, CD95, CD138, CS1/SLAMF7, KiR, Thy-1, Ly-6, Fas, APO-1, EGFR, HER2, CXCR4, HLA, GM1, and DRD.

In some embodiments, the multi-specific antibody is capable of binding to multiple identical or different tumor antigens.

In some embodiments, the multi-specific antibody is capable of binding to the same or different epitopes on the same tumor antigen.

In some embodiments, the multi-specific antibody is capable of specifically binding to HER2.

"HER2 (human epidermal growth factor receptor 2)" is encoded by the ErbB2 gene and is a member of the epidermal growth factor receptors (EGFR/ErbB). HER2 is known to play an pivotal role in regulating cell proliferation and differentiation. In particular, upon binding to extracellular growth factors, it exhibits a marked propensity to undergo homodimerization and/or a heterodimerization with other HER receptors, resulting in the activation of several forms of signal transduction pathways and the induction of apoptosis, survival or cell proliferation. For example, the HER2 protein can be a polypeptide deposited with GenBank accession number NP_004439.2, or NP_001005862.1, etc., which are encoded by nucleotide sequences (mRNA) deposited with GenBank accession numbers NM 004448.4, NM_001005862.3, etc., respectively.

The fragment that binds to HER2 and recognizes HER2 as an antigen can be selected from scFv, (scFv)₂, Fab, Fab' and F(ab')₂ of an anti-HER2 antibody.

In some embodiments, the multi-specific antibody comprises at least one (e.g., 1, 2, or 3) CDR of the heavy chain variable region of the anti-HER2 antibody, and/or at least one (e.g., 1, 2, or 3) CDR of the light chain variable region of the anti-HER2 antibody.

In some embodiments, the multi-specific antibody comprises the heavy chain variable region of the anti-HER2 antibody, and/or the light chain variable region of the anti-HER2 antibody.

In some embodiments, the anti-HER2 antibody is selected from trastuzumab, pertuzumab, and variants thereof.

In some embodiments, the variant has a substitution, deletion, or addition of one or more amino acids (e.g., a substitution, deletion, or addition of up to 20, up to 15, up to 10, or up to 5 amino acids; e.g., a substitution, deletion, or addition of 1, 2, 3, 4, or 5 amino acids) as compared to the wild-type sequence from which it is derived.

In some embodiments, the anti-HER2 antibody is a variant of trastuzumab. Specifically, the anti-HER2 antibody comprises the following 3 heavy chain variable region (VH) complementary determining regions (CDRs):
a VH CDR1 as set forth in SEQ ID NO: 46, a VH CDR2 as set forth in SEQ ID NO: 47, and a VH CDR3 as set forth in SEQ ID NO: 48; and/or,
the following 3 light chain variable region (VL) complementary determining regions (CDRs):
   a VL CDR1 as set forth in SEQ ID NO: 49, a VL CDR2 as set forth in SEQ ID NO: 50, and a VL CDR3 as set forth in SEQ ID NO: 8.

In some embodiments, the multi-specific antibody comprises a second antigen-binding domain specific for HER2.

In some embodiments, the first antigen-binding domain is VHH; the second antigen-binding domain is Fab, and the multi-specific antibody comprises:
(1) a peptide chain I-A, which comprises a light chain variable region and a light chain constant region (CL) of the second antigen-binding domain;
   and,
(2) a peptide chain I-B, which comprises a heavy chain variable region and a heavy chain constant region of the second antigen-binding domain, and the first antigen-binding domain; preferably, the peptide chain I-B comprises, from the N-terminal to the C-terminal, the adjacent heavy chain variable region and heavy chain constant region of the second antigen-binding domain, and the first antigen-binding domain.

In some embodiments, the heavy chain constant region can be selected from (e.g., Fc region): heavy chain constant regions of IgG1, IgG2, IgG3, and IgG4, specifically, heavy chain constant regions of human IgG1, IgG2, IgG3, or IgG4, for example, heavy chain constant regions of human IgG1, for example, CH1, CH2 and/or CH3 of human IgG1, and for example, Fc region of human IgG1.

In some embodiments, the heavy chain constant region is altered (e.g., mutated) to increase or decrease one or more of the following: Fc receptor binding, antibody glycosylation, number of cysteine residues, effector cell function, or complement function.

In some embodiments, the heavy chain constant region comprises a LALA mutation, a CH3 Knob mutation, a CH3 Hole mutation, a CH1/CL preference mutation CH SET1, a CH1/CL preference mutation CH SET2CH SET2, and any combination thereof.

In some embodiments, the heavy chain constant region is selected from the group consisting of:
HC-1: amino acid sequence as set forth in SEQ ID NO: 10;
HC-2: amino acid sequence as set forth in SEQ ID NO: 11;
HC-3: amino acid sequence as set forth in SEQ ID NO: 12;
HC-4: amino acid sequence as set forth in SEQ ID NO: 13;
HC-5: amino acid sequence as set forth in SEQ ID NO: 14;
HC-6: amino acid sequence as set forth in SEQ ID NO: 19;
HC-7: amino acid sequence as set forth in SEQ ID NO: 20;
HC-8: amino acid sequence as set forth in SEQ ID NO: 37; and
HC-9: amino acid sequence as set forth in SEQ ID NO: 38;
the variant has a substitution, deletion or addition of one or more amino acids as compared to the wild-type sequence from which it is derived (e.g., a substitution, deletion or addition of up to 20, up to 15, up to 10, or up to 5 amino acids; for example, a substitution, deletion or addition of 1, 2, 3, 4 or 5 amino acids).

In some embodiments, the light chain constant region is selected from the light chain constant region of κ or λ or a variant thereof;

The variant has a substitution, deletion or addition of one or more amino acids as compared to the wild-type sequence from which it is derived (e.g., a substitution, deletion or addition of up to 20, up to 15, up to 10, or up to 5 amino acids; for example, a substitution, deletion or addition of 1, 2, 3, 4 or 5 amino acids).

In some embodiments, the light chain constant region is selected from the group consistingof:
LC-1: SEQ ID NO: 9;
LC-2: SEQ ID NO: 15; and,
LC-3: SEQ ID NO: 16;
the variant has a substitution, deletion or addition of one or more amino acids as compared to the wild-type sequence from which it is derived (e.g., a substitution, deletion or addition of up to 20, up to 15, up to 10, or up to 5 amino acids; for example, a substitution, deletion or addition of 1, 2, 3, 4 or 5 amino acids).

In some embodiments, the CL of the peptide chain I-A is capable of forming a dimer with the heavy chain constant region CH1 domain of the peptide chain I-B.

In some embodiments, the multi-specific antibody comprises two peptide chains I-A and two peptide chains I-B; preferably, the heavy chain constant regions of the two peptide chains I-B form a dimer.

In some embodiments, the domains are connected directly or through a peptide linker.

In some embodiments, the peptide linker has a structure represented by (GₘXₙ)ₗG_{m'}, m, m', n and l are each independently selected from 0, 1, 2, 3, 4, 5, 6, 7, 8, 9 and 10, and X is selected from A and S; preferably, m is selected from 1, 2, 3, 4 and 5, n is selected from 1 and 2, l is selected from 1, 2, 3, 4, 5, 6, 7, 8, 9 and 10, m' is selected from 0 and 1, and X is selected from A and S.

In some embodiments, the peptide linker has an amino acid sequence as set forth in SEQ ID NO: 17 or 18.

In some embodiments, the multi-specific antibody is characterized by one or more of the following:
(i) the first antigen-binding domain comprises or consists of an amino acid sequence as set forth in SEQ ID NO: 7;
(ii) the heavy chain variable region of the second antigen-binding domain comprises or consists of an amino acid sequence as set forth in SEQ ID NO: 1, 3 or 5;
(iii) the light chain variable region of the second antigen-binding domain comprises or consists of an amino acid sequence as set forth in SEQ ID NO: 2, 4 or 6.

In some embodiments, the peptide chain I-A comprises or consists of an amino acid sequence as set forth in SEQ ID NO: 22, 24 or 26.

In some embodiments, the peptide chain I-B comprises or consists of an amino acid sequence as set forth in SEQ ID NO: 21, 23 or 25.

In some embodiments, the multi-specific antibody is selected from:
(1) a multi-specific antibody comprising the peptide chains I-A and I-B, wherein the peptide chain I-A comprise or consists of the amino acid sequence as set forth in SEQ ID NO: 22, and the peptide chain I-B comprises or consists of the amino acid sequence as set forth in SEQ ID NO: 21;
(2) a multi-specific antibody comprising the peptide chains I-A and I-B, wherein the peptide chain I-A comprises or consists of the amino acid sequence as set forth in SEQ ID NO: 24, and the peptide chain I-B comprises or consists of the amino acid sequence as set forth in SEQ ID NO: 23;
   and,
(3) a multi-specific antibody comprising the peptide chains I-A and I-B, wherein the peptide chain I-A comprises or consists of the amino acid sequence as set forth in SEQ ID NO: 26, and the peptide chain I-B comprises or consists of the amino acid sequence as set forth in SEQ ID NO: 25;

preferably, the multi-specific antibody comprises two peptide chains I-A and two peptide chains I-B;
preferably, the heavy chain constant regions of the two peptide chains I-B form a dimer.

In some embodiments, the multi-specific antibody further comprises a third antigen-binding domain specific for HER2.

In some embodiments, the first antigen-binding domain is VHH; the second antigen-binding domain and the second antigen-binding domain are Fab, and the multi-specific antibody comprises:
(1) a peptide chain II-A, which comprises a light chain variable region and a light chain constant region (CL) of the second antigen-binding domain;
(2) a peptide chain II-B, which comprises a heavy chain variable region and a heavy chain constant region of the second antigen-binding domain, and the first antigen-binding domain; preferably, the peptide chain II-B comprises, from the N-terminal to the C-terminal, the adjacent heavy chain variable region and heavy chain constant region of the second antigen-binding domain, and the first antigen-binding domain;
(3) a peptide chain II-C, which comprises a light chain variable region and a chain constant region (CL) of the third antigen-binding domain;
   and,
(4) a peptide chain II-D, which comprises a heavy chain variable region, a heavy chain constant region of the third antigen-binding domain, and the first antigen-binding domain; preferably, the peptide chain II-D comprises, from the N-terminal to the C-terminal, the adjacent heavy chain variable region and heavy chain constant region of the third antigen-binding domain, and the first antigen-binding domain.

In some embodiments, the heavy chain constant region can be selected from (e.g., Fc region): heavy chain constant regions of IgG1, IgG2, IgG3, and IgG4, specifically, heavy chain constant regions of human IgG1, IgG2, IgG3, or IgG4, for example, heavy chain constant regions of human IgG1, for example, CH1, CH2 and/or CH3 of human IgG1, and for another example, Fc region of human IgG1.

In some embodiments, the heavy chain constant region is altered (e.g., mutated) to increase or decrease one or more of the following: Fc receptor binding, antibody glycosylation, number of cysteine residues, effector cell function, or complement function.

In some embodiments, the heavy chain constant region comprises LALA mutation, CH3 Knob mutation, CH3 Hole mutation, CH1/CL preference mutation CH SET1, CH1/CL preference mutation CH SET2, and any combination thereof.

In some embodiments, the light chain constant region is selected from a light chain constant region of κ or λ or a variant thereof;

The variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived.

In some embodiments, the CL of the peptide chain II-A is capable of forming a dimer with the heavy chain constant region CH1 domain of the peptide chain II-B; preferably, the CL of the peptide chain II-C is capable of forming a dimer with the heavy chain constant region CH1 domain of the peptide chain II-D.

In some embodiments, the multi-specific antibody comprises one peptide chain II-A, one peptide chain II-B, one peptide chain II-C, and one peptide chain II-D. In some embodiments, the heavy chain constant regions of the peptide chains II-B and II-D form a dimer. In some embodiments, the heavy chain constant region of the peptide chain II-B comprises an amino acid sequence as set forth in SEQ ID NO: 13, and the heavy chain constant region of the peptide chain II-D comprises an amino acid sequence as set forth in SEQ ID NO: 14. In some embodiments, the heavy chain constant region of the peptide chain II-B comprises an amino acid sequence as set forth in SEQ ID NO: 37, and the heavy chain constant region of the peptide chain II-D comprises an amino acid sequence as set forth in SEQ ID NO: 38.

In some embodiments, the domains are connected directly or through a peptide linker.

In some embodiments, the peptide linker has a structure represented by (GₘXₙ)ₗG_{m'}, wherein m, m', n and l are each independently selected from 0, 1, 2, 3, 4, 5, 6, 7, 8, 9 and 10, and X is selected from A and S.

In some embodiments, m is selected from 1, 2, 3, 4 anda 5, n is selected from 1 and 2, l is selected from 1, 2, 3, 4, 5, 6, 7, 8, 9 and 10, m' is selected from 0 and 1, and X is selected from A and S.

In some embodiments, the peptide linker has an amino acid sequence as set forth in SEQ ID NO: 17 or 18.

In some embodiments, the multi-specific antibody is characterized by one or more of the following features:
(i) the first antigen-binding domain comprises or consists of an amino acid sequence as set forth in SEQ ID NO: 7;
(ii) the heavy chain variable region of the second antigen-binding domain comprises or consists of an amino acid sequence as set forth in SEQ ID NO: 1, 3 or 5;
(iii) the light chain variable region of the second antigen-binding domain comprises or consists of an amino acid sequence as set forth in SEQ ID NO: 2, 4 or 6;
(iv) the heavy chain variable region of the third antigen-binding domain comprises or consists of an amino acid sequence as set forth in SEQ ID NO: 1, 3 or 5;
(v) the light chain variable region of the third antigen-binding domain comprises or consists of an amino acid sequence as set forth in SEQ ID NO: 2, 4 or 6.

In some embodiments, the peptide chain II-A comprises or consists of an amino acid sequence as set forth in SEQ ID NO: 28 or 40.

In some embodiments, the peptide chain II-B comprises or consists of an amino acid sequence as set forth in SEQ ID NO: 27 or 39.

In some embodiments, the peptide chain II-C comprises or consists of an amino acid sequence as set forth in SEQ ID NO: 30 or 42.

In some embodiments, the peptide chain II-D comprises or consists of an amino acid sequence as set forth in SEQ ID NO: 29 or 41.

In some embodiments, the multi-specific antibody is selected from:
(1) a multi-specific antibody comprising the peptide chains II-A, II-B, II-C and II-D, wherein the peptide chain II-A comprises or consists of an amino acid sequence as set forth in SEQ ID NO: 28, the peptide chain II-B comprises or consists of an amino acid sequence as set forth in SEQ ID NO: 27, the peptide chain II-C comprises or consists of an amino acid sequence as set forth in SEQ ID NO: 29, and the peptide chain II-D comprises or consists of an amino acid sequence as set forth in SEQ ID NO: 30;
   and,
(2) a multi-specific antibody comprising the peptide chains II-A, II-B, II-C and II-D, wherein the peptide chain II-A comprises or consists of an amino acid sequence as set forth in SEQ ID NO: 40, the peptide chain II-B comprises or consists of an amino acid sequence as set forth in SEQ ID NO: 39, the peptide chain II-C comprises or consists of an amino acid sequence as set forth in SEQ ID NO: 42, and the peptide chain II-D comprises or consists of an amino acid sequence as set forth in SEQ ID NO: 41.

### Peptide linker

The N-terminal or C-terminal (preferably N-terminal) of the part binding to 4-1BB as described herein is linked directly or through a peptide linker to the C-terminal or N-terminal of the heavy chain constant region.

As used herein, the term "peptide linker" may refer to an oligopeptide comprising 1 to 100 amino acids, in particular 2 to 50 amino acids, and each of the amino acids may be any kind of amino acid without any limitation. Any conventional peptide linker may be used with or without appropriate modification to meet a specific purpose. In a specific embodiment, the peptide linker may contain, for example, Gly, Asn and/or Ser residues, and/or contain neutral amino acids such as Thr and/or Ala. The amino acid sequence suitable for the peptide linker may be known in the relevant art. The length of the peptide linker may be appropriately determined within limits that do not affect the function of the polypeptide and/or scFv. For example, the peptide linker can be formed by including a total of about 1 to about 100 amino acids, about 2 to about 50 amino acids, or about 5 to about 25 (e.g., 1, 2, 3, 4, 5, 6, 7, 8, 9, 10, 11, 12, 13, 14, 15, 16, 17, 18, 19, 20, 21, 22, 23, 24, or 25) amino acids, and each of the amino acids is independently selected from the group consisting of Gly, Asn, Ser, Thr, and Ala.

In some embodiments, the peptide linker has a structure shown in (GₘXₙ)ₗG_{m'}, m, m', n, and l are each independently selected from 0, 1, 2, 3, 4, 5, 6, 7, 8, 9, and 10, and X is selected from A and S.

In some embodiments, m is selected from 1, 2, 3, 4 and 5, n is selected from 1 and 2, l is selected from 1, 2, 3, 4, 5, 6, 7, 8, 9 and 10, m' is selected from 0 and 1, and X is selected from A and S.

In some embodiments, the peptide linker has an amino acid sequence as set forth in SEQ ID NO: 17 or 18.

### Preparation of polynucleotide, recombinant vector and antibody

In one aspect, the present invention provides a polynucleotide, which encodes the antibody or antigen-binding fragment thereof as described in any one of the above items.

In one aspect, the present invention provides a vector, which comprises the polynucleotide as described above.

In one aspect, the present invention provides a recombinant cell, which comprises the polynucleotide or vector as described above. The recombinant cell may be a cell transfected with a recombinant vector.

In one aspect, the present invention provides a method for preparing the antibody or antigen-binding fragment thereof, comprising expressing a polynucleotide in a cell. The step of expressing the polynucleotide may be performed by culturing a cell comprising the polynucleotide (e.g., the polynucleotide is in a recombinant vector) under conditions that allow the expression of the polynucleotide. The method may further comprise isolating and/or purifying the antibody or antigen-binding fragment thereof from the cell culture after the expression or culturing step.

### Application

The present invention further provides a use of the antibody or antigen-binding fragment thereof as described herein in enhancing immune response and/or treating a tumor.

Specifically, the present invention provides a pharmaceutical composition, which comprises the antibody or antigen-binding fragment thereof, polynucleotide, vector or recombinant cell as described in any one of the above items, and a pharmaceutically acceptable excipient.

In another aspect, the present invention further provides a use of the antibody or antigen-binding fragment thereof, polynucleotide, vector, recombinant cell or pharmaceutical composition as described in any one of the above items in the manufacture of an anti-tumor medicament.

In another aspect, the present invention provides an anti-tumor method, which comprises a step of administering a therapeutically effective amount of the antibody or antigen-binding fragment thereof, polynucleotide, vector, recombinant cell or pharmaceutical composition as described in any one of the above items to a subject in need thereof.

In some embodiments, the tumor overexpresses HER2.

In some embodiments, the tumor is selected from the group consisting of breast cancer, colon cancer, stomach cancer, lung cancer (e.g., squamous cell lung cancer, small cell lung cancer, non-small cell lung cancer, lung adenocarcinoma), peritoneal cancer, skin cancer, squamous cell carcinoma, melanoma of skin or eyeball, rectal cancer, cancer near anus, esophageal cancer, small intestine tumor, endocrine gland cancer, parathyroid cancer, adrenal cancer, soft tissue sarcoma, urethral cancer, chronic or acute leukemia, lymphocytic lymphoma, liver cancer, gastrointestinal cancer, pancreatic cancer, glioblastoma, cervical cancer, ovarian cancer, liver cancer, bladder cancer, hepatocellular adenoma, colorectal cancer, endometrial cancer or uterine cancer, salivary gland tumor, kidney cancer, cervical cancer, prostate cancer, vulvar cancer, thyroid cancer, head and neck cancer, brain cancer, biliary tract cancer and gallbladder cancer.

In some embodiments, the tumor is a primary or metastatic tumor.

### Detection use

In another aspect, the present invention also provides a conjugate, which comprises the antibody or antigen-binding fragment thereof or the polypeptide construct as described in any one of the above aspects, and a detectable label attached to the single-domain antibody or antigen-binding fragment thereof or the polypeptide construct.

In some embodiments, the detectable label is selected from the group consisting of enzyme (e.g., horseradish peroxidase or alkaline phosphatase), chemiluminescent agent (e.g., an acridinium ester, luminol and a derivative thereof, or a ruthenium derivative), fluorescent dye (e.g., fluorescein or fluorescent protein), radionuclide or biotin.

On the other hand, the present invention also provides a kit, which comprises the single-domain antibody or antigen-binding fragment thereof or the polypeptide construct, or the conjugate as described in any one of the above aspects.

In certain embodiments, the kit further comprises a second antibody capable of specifically recognizing an antigen specifically recognized by the antibody or antigen-binding fragment thereof or the polypeptide construct; optionally, the second antibody further comprises a detectable label, such as an enzyme (e.g., horseradish peroxidase or alkaline phosphatase), a chemiluminescent agent (e.g., an acridinium ester, luminol and a derivative thereof, or a ruthenium derivative), a fluorescent dye (e.g., fluorescein or fluorescent protein), a radionuclide or biotin.

In some embodiments, the second antibody targets the same or different antigenic epitopes as the antibody or antigen-binding fragment thereof or the polypeptide construct.

On the other hand, the present invention also provides a method for detecting the presence or level of 4-1BB in a sample, which comprises using the antibody or antigen-binding fragment thereof or the polypeptide construct, or the conjugate as described in any one of the above aspects.

In certain embodiments, the method is an immunological assay, such as immunoblotting, enzyme immunoassay (e.g., ELISA), chemiluminescent immunoassay, fluorescent immunoassay, or radioimmunoassay.

In certain embodiments, the method comprises using the conjugate as described above.

In certain embodiments, the method comprises using the antibody or antigen-binding fragment thereof or the polypeptide construct as described in any one of the above aspects, and the method further comprises using a second antibody carrying a detectable label (e.g., an enzyme (e.g., horseradish peroxidase or alkaline phosphatase), a chemiluminescent agent (e.g., an acridinium ester, luminol and a derivative thereof, or a ruthenium derivative), a fluorescent dye (e.g., fluorescein or fluorescent protein), a radionuclide, or biotin) to detect the binding of the single-domain antibody or antigen-binding fragment thereof or the polypeptide construct to the antigen.

In certain embodiments, the method comprises: (1) contacting the sample with the antibody or antigen-binding fragment thereof; (2) detecting the formation of an antigen-antibody immune complex or detecting the amount of the immune complex. The formation of the immune complex indicates the presence of 4-1BB or a cell expressing 4-1BB.

On the other hand, the present application also provides a use of the single-domain antibody or antigen-binding fragment thereof, or the polypeptide construct, or the conjugate as described in any one of the above aspects in the manufacture of a detection reagent, wherein the detection reagent is used to detect the presence or level of 4-1BB in a sample.

In certain embodiments, the detection reagent detects the presence or level of 4-1BB in the sample by the method for detecting the presence or level of 4-1BB in a sample as described above.

In certain embodiments, the sample is a cell sample (e.g., a tumor cell) from a subject (e.g., a mammal, preferably a human or a monkey).

### Definitions

As used herein, unless otherwise specified, the scientific and technical terms used herein have the meanings commonly understood by those skilled in the art. In addition, the protein and nucleic acid chemistry, molecular biology, cell and tissue culture, microbiology, immunology and related terms and laboratory operation steps used herein are all terms and routine steps widely used in the corresponding fields. At the same time, in order to better understand the present invention, the definitions and explanations of the relevant terms are provided below.

As used herein, "at least one (kind)" or "one (kind) or more (kinds)" may refer to 1, 2, 3, 4, 5, 6, 7, 8, 9, 10, 11, 12, 13, 14, 15, 16, 17, 18, 19, 20 (kinds) or more (kinds).

The expression "comprising" or its synonymous or similar expressions "including", "containing" and "having" are open and do not exclude additional unlisted elements, steps or components. The expression "consisting of ..." excludes any unspecified elements, steps or components. The expression "consisting of ..." means that the scope is limited to the specified elements, steps or components, plus the optional elements, steps or components that do not substantially affect the basic and new features of the subject matter sought to be protected. It should be understood that the expression "comprising" encompasses the expressions "consisting essentially of ..." and "consisting of ...".

The term "optionally" or "optional" means that the subsequently described event or situation may or may not occur, and the description comprises the occurrence of the event or situation and the non-occurrence of the event or situation.

As used herein, "peptide", "polypeptide" refers to a polymer formed by two or more amino acids linked by peptide bonds. "Protein" can be formed by one or more polypeptides in a covalent or non-covalent manner. Unless otherwise specified, the terms "polypeptide" and "protein" are used interchangeably herein.

As used herein, "antibody" refers to an immunoglobulin or fragment thereof, which specifically binds to an antigen epitope through at least one antigen binding site. As used herein, the definition of antibody encompasses antigen binding fragments. Therefore, the term "antibody" includes multi-specific antibodies (e.g., bispecific antibodies), human antibodies, non-human antibodies, humanized antibodies, chimeric antibodies, single domain antibodies, and antigen binding fragments. Antibodies can be produced by synthesis (e.g., produced by chemical coupling or biological coupling), enzymatic treatment, or recombination. The antibodies provided herein include any immunoglobulin type (e.g., IgG, IgM, IgD, IgE, IgA, and IgY), any class (e.g., IgG1, IgG2, IgG3, IgG4, IgA1, and IgA2), or subclass (e.g., IgG2a and IgG2b). In one embodiment, the antibody of the present invention is a murine monoclonal antibody. In another embodiment, the antibody of the present invention is a humanized monoclonal antibody.

As used herein, "traditional antibody" or "full-length antibody" generally comprises four polypeptides: two heavy chains (HC) and two light chains (LC). Each light chain comprises a "light chain variable region (VL)" and a "light chain constant region (CL)" from the N-terminal to the C-terminal. Each heavy chain comprises a "heavy chain variable region (VH)" and a "heavy chain constant region (CH)" from the N-terminal to the C-terminal. The heavy chain constant region may comprise CH1, CH2, and CH3 from the N-terminal to the C-terminal. In some immunoglobulin types (e.g., IgM and IgE), the heavy chain constant region may also comprise CH4. "Fc" fragment refers to a fragment comprising CH2 and CH3, which provides a site for binding to Fc receptor. "Hinge region" refers to a part of antibody that connects Fab and Fc fragments of immunoglobulin. In some cases, hinge region refers to a part of T cell receptor that connects constant region and transmembrane domain. As used herein, when used for chimeric antigen receptors, the hinge region may also refer to any functional equivalent. Those skilled in the art can determine the positions of VH, VL, CL, CH1, CH2, CH3 and hinge region in the antibody according to known algorithms and software. The description of algorithms and software that can be applied can be found in, for example, William R. Strohl, Lila M. Strohl, (2012), Antibody structure-function relationships, In Woodhead Publishing Series in Biomedicine, Therapeutic Antibody Engineering, Woodhead Publishing, pp. 37-56.

Typically, VL and VH each may comprise three highly variable "complementarity determining regions (CDRs)" and four relatively conservative "framework regions (FRs)", and are connected in the sequence of FR1-CDR1-FR2-CDR2-FR3-CDR3-FR4 from the N-terminal to the C-terminal. As used herein, the light chain variable region CDR (CDRL) may be referred to as CDR-L1, CDR-L2, and CDR-L3, and the heavy chain variable region CDR (CDRH) may be referred to as CDR-H1, CDR-H2, and CDR-H3. It is generally believed that the six CDRs comprising a pair of VH and VL determine the binding specificity of antigen binding site; but in some cases, other fragments (e.g., single domain antibodies, also known as nanobodies) comprising less than six (e.g., three, four, or five) CDRs also have the ability to bind to antigens. Those skilled in the art may identify CDRs using methods well known in the art, such as using Kabat, Abm, or Chothia numbering method. As used herein, multiple CDR numbering systems, such as Chothia, Abm, Kabat, and IMGT, may be used for the same variable region. Those skilled in the art will appreciate that, although the CDRs defined by different numbering systems may be different, the CDRs corresponding to the same numbering system represent effective antigen binding sites capable of binding to antigen epitopes. For the description of CDR numbering systems, see, for example, the Kabat numbering system: Kabat, E.A. et al. (1991) Sequences of Proteins of Immunological Interest, Fifth Edition, U.S. Department of Health and Human Services, NIH Publication No. 91-3242; the Chothia numbering system: Chothia, C. et al. (1987) J. Mol. Biol. 196:901-917; the IMGT numbering system: Lefranc, M.-P., 2011(6), IMGT, the International ImMunoGeneTics Information System Cold Spring Harb Protoc.; the Abm numbering system: Martin, A.C.R. and J. Allen (2007) "Bioinformatics tools for antibody engineering," in S. Dübel (ed.), Handbook of Therapeutic Antibodies. Weinheim: Wiley-VCH Verlag, pp. 95-118.

As used herein, the terms "framework region" and "framework" can be used interchangeably. As used herein, the terms "framework region", "framework" or "FR" residues refer to those amino acid residues in a variable region of an antibody other than the CDR residues as defined above.

It is generally believed that the "Fv" fragment composed of a VH and a VL through non-covalent interaction is the smallest antigen-binding fragment containing an antigen-binding site. However, a single-domain antibody (VHH, also known as sdAb or nanobody) containing only heavy chain variable region can also have antigen binding ability. VH and VL can be connected by a peptide linker to obtain a "single-chain Fv (scFv)". By introducing disulfide bonds into Fv or scFv, "disulfide-stabilized Fv (dsFv)" or "single-chain disulfide-stabilized Fv (scdsFv or dsscFv)" can be obtained respectively. In some embodiments, for example, the antigen-binding fragment in the single-domain antibody, bispecific antibody, and multi-specific antibody of the present invention preferably uses scFv or scdsFv.

As used herein, "Fab" comprises one intact antibody light chain (VL-CL) and antibody heavy chain variable region and one heavy chain constant region (VH-CH1, also referred to as Fd). Single-chain "Fab (scFab)" can be obtained by connecting CL and CH1 in "Fab" with a peptide linker. "F(ab')₂" basically comprises two Fab fragments connected by a disulfide bond in the hinge region. "Fab'" is half of F(ab')₂, which can be obtained by reducing the disulfide bond in the hinge region of F(ab')₂.

Antibodies or antigen-binding fragments can be "monovalent", "divalent", "trivalent" or "tetravalent" or "multivalent", which mean that they have a plurality of (e.g., 1, 2, 3 or 4 or more) antigen binding sites.

When an antibody or antigen-binding fragment binds to two or more (e.g., 2, 3, 4, 5 or 6) antigens, it can also be referred to as a "multi-specific antibody", such as a bispecific antibody, a trispecific antibody or a tetraspecific antibody, which respectively represent a multi-specific antibodies capable of binding to 2, 3 or 4 antigens.

As used herein, "diabody" refers to an antibody comprising two scFvs, having two antigen binding sites (divalent), wherein the VH and VL in each scFv are connected by a short peptide linker (about 5-10 amino acid residues), so that the VH and VL chains are paired (i.e., the VH of the first scFv and the VL of the second scFv are paired, and the VL of the first scFv and the VH of the second scFv are paired) to form an antigen binding site. A diabody can be a bispecific antibody.

As used herein, an "antigen-binding fragment" of an antibody refers to a portion of a full-length antibody that is less than the full length, but at least comprises a portion of the variable region of the full-length antibody (e.g., comprises one or more CDRs and/or one or more antigen binding sites), and thus retains at least part of the ability of the full-length antibody to specifically bind to an antigen. Antigen-binding fragments can, for example, include antibody derivatives produced by enzymatic treatment of full-length antibodies, synthetically produced derivatives, and recombinantly produced derivatives. Examples of antigen-binding fragments include, but are not limited to, Fv, scFv, dsFv, scdsFv, Fab, scFab, Fab', F(ab')₂, diabody, Fd and Fd' fragments, and other fragments (e.g., fragments containing modifications). An antigen-binding fragment may comprise multiple peptide chains, for example, connected by a disulfide bond and/or peptide linker and/or formed by non-covalent interaction.

As used herein, the term "monoclonal antibody" refers to a group of highly homogeneous antibodies, wherein the antibody molecules contained therein are substantially identical except for possible naturally occurring mutations that may be present in small amounts. Monoclonal antibodies generally specifically bind to a single antigenic epitope. The monoclonal antibody as used herein can be prepared by any method known in the art, for example, produced from transgenic animals (e.g., transgenic mice), produced by immortalized B cells (e.g., B cell hybridomas), or prepared in bacteria, eukaryotic animals or plant cells using recombinant DNA methods, or isolated from phage antibody libraries. The antibody or antigen-binding fragment of the present invention is a monoclonal antibody. In one embodiment, the antibody or antigen-binding fragment of the present invention is a humanized monoclonal antibody.

"Chimeric antibody" refers to an antibody in which a portion (e.g., CDR, FR, variable region, constant region, or combination thereof) is identical or homologous to the corresponding sequence in an antibody derived from a particular species, and the remaining portion is identical or homologous to the corresponding sequence in an antibody derived from another species. As used herein, "chimeric antibody" also covers antibodies that contain portions belonging to different antibody types or subclasses. In a specific embodiment, the chimeric antibody has a murine antibody variable region and a human antibody constant region.

As used herein, "humanized antibody" refers to an antibody containing non-human antibody and human antibody sequences. Therefore, a humanized antibody is a chimeric antibody containing minimal sequence derived from a non-human immunoglobulin. A non-human antibody may be an antibody derived from any non-human species or an antibody containing a portion of a non-human species (e.g., a chimeric antibody). Non-human species may include, for example, mice, rats, rabbits, alpacas, or non-human primates. The techniques for obtaining humanized antibodies from non-human antibodies are well known to those skilled in the art. Humanized antibodies may be produced from non-human antibodies (e.g., murine antibodies or chimeric antibodies), for example, by transplanting a CDR sequence of a non-human antibody (e.g., murine antibody) into a human antibody framework region. In some cases, in order to maintain the antigen binding ability and/or stability of a humanized antibody, the key amino acid residues of a framework sequence of a non-human antibody (e.g., murine antibody) may be retained in the human antibody framework region, i.e., a "back mutation" may be performed (see, for example, Morrison et al. (1984) Proc. Natl. Acad. Sci. 81(21): 6851-6855; Neuberger et al. (1984) Nature 312: 604-608).

As used herein, "percent (%) sequence identity" and "sequence identity" of amino acid sequences have definitions well known in the art, which refer to the percentage of identity between two polypeptide sequences determined by sequence alignment (e.g., by manual inspection or well-known algorithms). It can be determined using methods known to those skilled in the art, such as using publicly available computer software such as BLAST, BLAST-2, Clustal Omega, and FASTA software.

"Affinity" or "binding affinity" is used to measure the strength of mutual binding between an antibody and an antigen through non-covalent interactions. The magnitude of "affinity" can usually be reported as equilibrium dissociation constant K_{D} or EC₅₀. K_{D} can be calculated by the measured equilibrium association constant (ka) and equilibrium dissociation constant (kd): K_{D} = kd/ka. Affinity can be determined using conventional techniques known in the art, such as biomembrane interferometry (e.g., Octet Fortebio detection system can be used), radioimmunoassay, surface plasmon resonance, enzyme-linked immunosorbent assay, or flow cytometry.

As used herein, "specific binding" between an antibody and an antigen means that the antibody and the antigen are bound to each other with a higher affinity. Typically, the K_{D} value between an antibody and an antigen that are specifically bound is at least about 10⁻⁶ to at least about 10⁻⁹ M or lower, for example, at least about 10⁻⁶, at least about 10⁻⁷, at least about 10⁻⁸, at least about 10⁻⁹ M or lower.

As used herein, an amino acid sequence "from" or "derived from" a reference amino acid sequence is partially or completely identical or homologous to the reference amino acid sequence. For example, an amino acid sequence derived from a heavy chain constant region of human immunoglobulin has a sequence identity of at least 80%, at least 85%, at least 90%, at least 91%, at least 92%, at least 93%, at least 94%, at least 95%, at least 96%, at least 97%, at least 98%, at least 99% or 100% as compared to the wild-type sequence of the heavy chain constant region of human immunoglobulin from which it is derived.

As used herein, a "variant" of a polypeptide or amino acid sequence has one or more amino acid mutations or modifications as compared to the polypeptide or amino acid sequence from which it is derived. Amino acid mutations include substitutions, deletions or additions of amino acids. Those skilled in the art will appreciate that amino acids in non-essential regions of polypeptides or proteins can be substituted with suitable conservative amino acids, and such substitution generally does not change their biological activity (see, for example, Watson et al., Molecular Biology of the Gene, 4th Edition, 1987, The Benjamin/Cummings Pub. co., p.224). Suitable conservative substitutions are well known to those skilled in the art. The following table lists some non-limiting examples of common conservative substitutions of amino acid residues. In some cases, amino acid substitutions are non-conservative substitutions. Those skilled in the art will appreciate that amino acid mutations or modifications can be made to antibodies or antibody fragments to change their properties, for example, to change their antibody glycosylation modification type, and to change their ability to form interchain disulfide bonds. Antibodies or antigen-binding fragments thereof containing such amino acid mutations or modifications are also covered within the scope of the antibody or antigen-binding fragment thereof of the present invention.

| Original residue | Exemplary conservative substitutions | Preferred substitution |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe | Leu |
| Leu (L) | Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala | Leu |

As used herein, the terms "polynucleotide", "nucleic acid" and "nucleic acid molecule" refer to an oligomer or polymer comprising at least two linked nucleotides or nucleotide derivatives, and may generally comprise deoxyribonucleic acid (DNA) and ribonucleic acid (RNA).

As used herein, "isolated" means that a substance (e.g., a nucleic acid molecule or polypeptide) is separated from its source or environment, i.e., it does not substantially contain any other components.

As used herein, "vector" is a medium for introducing an exogenous nucleic acid into a host cell, and when the vector is transformed into an appropriate host cell, the exogenous nucleic acid is amplified or expressed. The vector is usually kept free, but can be designed to allow the gene or part thereof to be integrated into the chromosome of genome. As used herein, the definition of vector encompasses plasmids, linearized plasmids, viral vectors, cosmids, phage vectors, phagemids, artificial chromosomes (e.g., yeast artificial chromosomes and mammalian artificial chromosomes), and the like.

As used herein, "expression vector" refers to a vector capable of expressing DNA, in which the DNA is operably linked to a regulatory sequence (e.g., a promoter, a ribosome binding site) that can affect DNA expression. The regulatory sequence may comprise promoter and terminator sequences, and may optionally comprises an origin of replication, a selection marker, an enhancer, a polyadenylation signal, and the like. The expression vector may be a plasmid, a phage vector, a recombinant virus, or other vector that, when introduced into an appropriate host cell, results in the expression of the cloned DNA. Suitable expression vectors are well known to those skilled in the art and include expression vectors that are replicable in eukaryotic cells and/or prokaryotic cells, as well as expression vectors that remain free or that are integrated into the host cell genome.

As used herein, "recombinant cell" is a cell that is used to receive, maintain, replicate, or amplify a vector. Recombinant cell may also be used to express a polypeptide encoded by a nucleic acid or vector. Recombinant cell may be an eukaryotic cell or a prokaryotic cell.

As used herein, the term "treatment" refers to improvement of a disease/symptom, such as alleviation or elimination of the disease/symptom, prevention or slowing down of the occurrence, progression and/or worsening of the disease/symptom. Therefore, treatment comprises prevention, treatment and/or cure.

"Effective amount" refers to a dose that is sufficient to reduce the severity of a disease symptom, increase the frequency and duration of disease-free period, or prevent damage or disability caused by the disease. "Effective amount" refers to an amount required to prevent, cure, improve, block or partially block a disease or symptom. For example, for the treatment of tumors, an "effective amount" of the antibody or antigen-binding fragment thereof, or pharmaceutical composition of the present invention preferably inhibits tumor cell growth or tumor growth by at least about 10%, preferably at least about 20%, more preferably at least about 30%, more preferably at least about 40%, more preferably at least about 50%, more preferably at least about 60%, more preferably at least about 70%, and more preferably at least about 80%, relative to an untreated subject. The efficacy of inhibiting tumor growth can be evaluated using conventional tumor animal models in the art, such as spontaneous tumor, induced tumor, and transplanted tumor animal models. Alternatively, the ability to inhibit cell growth can also be examined using *in vitro* detection methods known in the art. An effective amount of the antibody or antigen-binding fragment thereof, or pharmaceutical composition of the present invention can reduce tumor size, or otherwise alleviate a symptom of subject (e.g., prevent and/or treat metastasis or recurrence). A person skilled in the art can determine the effective amount based on factors such as the age, physical condition, gender, severity of symptom of the subject, as well as specific composition or administration route. The effective amount can be given in one or more administrations.

As used herein, the term "pharmaceutically acceptable excipient" refers to an excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and includes, but is not limited to: a pH regulator, a surfactant, an adjuvant, an ionic strength enhancer, a diluent, an agent for maintaining osmotic pressure, an agent for delaying absorption, a preservative. For example, the pH regulator includes, but is not limited to, phosphate buffer. The surfactant includes, but is not limited to, cationic, anionic or non-ionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and analog thereof. The agent for delaying absorption includes, but is not limited to, monostearate and gelatin. The diluent includes, but is not limited to, water, aqueous buffer (e.g., buffered saline), alcohol, and polyol (e.g., glycerol), etc. The preservative includes, but is not limited to, various antibacterial and antifungal agents, such as thimerosal, 2-phenoxyethanol, paraben, chlorobutanol, phenol, sorbic acid, etc. The stabilizer has the meaning commonly understood by those skilled in the art, and is capable of stabilizing the desired activity of an active ingredient in a drug, including, but not limited to, sodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin, or casein), or degradation product thereof (e.g., lactalbumin hydrolysate), etc.

As used herein, examples of mammals include, but are not limited to, humans, non-human primates, rats, mice, cattle, horses, pigs, sheep, dogs, cats, etc. As used herein, the term "subject" refers to a mammal, such as a human. In some embodiments, the subject is a human. In some embodiments, the subject is a cancer patient, a human or animal suspected of having cancer or at risk of cancer. As used herein, the term "patient" and "subject" can be used interchangeably.

### Brief Description of the Drawings

The drawings described herein are used to provide a further understanding of the present invention and constitute a part of the present application. The exemplary examples of the present invention and their descriptions are used to explain the present invention and do not constitute an improper limitation of the present invention. In the drawings:
Figure 1 shows a schematic diagram of the structure of the multi-specific antibody as described in the present application.
Figure 2 shows the binding of the multi-specific antibody of the present invention to human HER2 as detected on cells N87 and CT26 overexpressing HER2.
Figure 3 shows the binding of the multi-specific antibody of the present invention to human 4-1BB as detected on cells CHO-h4-1BB overexpressing human 4-1BB.
Figure 4 shows the binding of the multi-specific antibody of the present invention to FcR.
Figure 5 shows that the multi-specific antibody of the present invention blocks the proliferation of HER2 signal-dependent cells N87 or SK-OV-3.
Figure 6 shows that the multi-specific antibody of the present invention mediates ADCC function through HER2.
Figure 7 shows that HER2 mediates the release of cytokines IL-2 and IFN-γ by primary T cells induced by the multi-specific antibody of the present invention.
Figure 8 shows that FcR mediates the release of cytokines IL-2 and IFN-γ by primary T cells induced by the multi-specific antibody of the present invention.
Figure 9 shows the SEC and LC/MS detection results of the multi-specific antibody HER2xHER2x4-1BB of the present invention.
Figure 10 shows the tumor inhibitory activity of the multi-specific antibody of the present invention in a tumor model of h-4-1BB KI BALB/c mice subcutaneously inoculated with CT26-h-HER2, wherein Figure 10A shows the effect on tumor volume 0 to 15 days after administration, and Figure 10B shows the CD8+T cell infiltration in mouse tumors.
Figure 11 shows the tumor inhibitory activity of the multi-specific antibody of the present invention in a tumor model of h-4-1BB KI BALB/c mice subcutaneously inoculated with CT26-h-HER2 (the effect on tumor volume 11 to 27 days after administration).
Figure 12 shows the anti-tumor activity of the multi-specific antibody of the present invention in a tumor model of h-4-1BB KI C57 mice subcutaneously inoculated with MC38-h-HER2.

### Specific Models for Carrying Out the present invention

The technical solution of the present invention will be described clearly and completely in conjugation with the examples and drawings of the present invention. Obviously, the described examples are only part of the examples of the present invention, not all of them. The following description of at least one exemplary example is actually only illustrative and is by no means a limitation on the present invention and its application or use. Based on the examples of the present invention, all other examples obtained by an ordinary skilled person in the art without creative work are within the scope of the present invention.

### Summary of sequence information

| SEQ ID | Description |
|---|---|
| NO: | |
| 1 | Heavy chain variable region of anti-Trastuzumab mAb |
| 2 | Light chain variable region of anti-Trastuzumab mAb |
| 3 | Heavy chain variable region of anti-Pertuzumab mAb |
| 4 | Light chain variable region of anti-Pertuzumab mAb |
| 5 | Heavy chain variable region of anti-HER2 mAb |
| 6 | Light chain variable region of anti-HER2 mAb |
| 7 | 4-1BB binding region of anti-4-1BB nanobody AB24 ME |
| 8 | anti-HER2 mAb VLCDR3 |
| 9 | Amino acid sequence of human κ light chain constant region (CL) |
| 10 | Amino acid sequence of human IgG1 |
| 11 | Amino acid sequence of human IgG1 (introduced with LALA mutation to reduce Fc function) |
| 12 | Amino acid sequence of human IgG1 |
| 13 | CH1/CL preference-mutated human IgG1 Fc amino acid sequence CH SET1 (introduced with Knob mutation) |
| 14 | CH1/CL preference-mutated human IgG1 Fc amino acid sequence CH SET2 (introduced with Hole mutation) |
| 15 | CH1/CL preference-mutated human κ light chain constant region (CL) amino acid sequence CL SET1 |
| 16 | CH1/CL preference-mutated human κ light chain constant region (CL) amino acid sequence CL SET2 |
| 17 | (G4S)4G |
| 18 | (G4A)4 |
| 19 | Amino acid sequence of human IgG1 Fc |
| 20 | Amino acid sequence of IgG1 Fc (introduced with LALA mutation to reduce Fc function) |
| 21 | HER2x4-1BB-1 heavy chain (HER2x4-1BB-1 peptide chain #1) |
| 22 | HER2x4-1BB-1 light chain (HER2x4-1BB-1 peptide chain #2) |
| 23 | HER2x4-1BB-2 heavy chain (HER2x4-1BB-2 peptide chain #1) |
| 24 | HER2x4-1BB-2 light chain (HER2x4-1BB-2 peptide chain #2) |
| 25 | HER2x4-1BB-3 heavy chain (HER2x4-1BB-3 peptide chain #1) |
| 26 | HER2x4-1BB-3 light chain (HER2x4-1BB-3 peptide chain #2) |
| 27 | HER2x HER2x4-1BB-1 heavy chain 1 (HER2xHER2x4-1BB-1 peptide chain #1) |
| 28 | HER2x HER2x4-1BB-1 light chain 1 (HER2xHER2x4-1BB-1 peptide chain #2) |
| 29 | HER2x HER2x4-1BB-1 heavy chain 2 (HER2xHER2x4-1BB-1 peptide chain #3) |
| 30 | HER2x HER2x4-1BB-1 light chain2 (HER2xHER2x4-1BB-1 peptide chain #4) |
| 31 | Anti-4-1BB V⁻HH-1 |
| 32 | Anti-4-1BB VHH-2 |
| 33 | Urelumab heavy chain (Urelumab peptide chain #1) |
| 34 | Urelumab light chain (Urelumab peptide chain #2) |
| 35 | PRS-343 heavy chain (PRS-343 peptide chain #1) |
| 36 | PRS-343 light chain (PRS-343 peptide chain #2) |
| 37 | Human IgG1 amino acid sequence HC-1 |
| 38 | Human IgG1 amino acid sequence HC-2 |
| 39 | HER2x HER2x4-1B-2 heavy chain 1 (HER2xHER2x4-1BB-2 peptide chain #1) |
| 40 | HER2x HER2x4-1B-2 light chain 1 (HER2xHER2x4-1BB-2 peptide chain #2) |
| 41 | HER2x HER2x4-1BB-2 heavy chain 2 (HER2xHER2x4-1BB-2 peptide chain #3) |
| 42 | HER2x HER2x4-1BB-2 light chain 2 (HER2xHER2x4-1BB-2 peptide chain #4) |
| 43 | CDR1 of anti-4-1BB nanobody AB24 ME |
| 44 | CDR2 of anti-4-1BB nanobody AB24 ME |
| 45 | CDR3 of anti-4-1BB nanobody AB24 ME |
| 46 | Anti-HER2 mAb VHCDR1 |
| 47 | Anti-HER2 mAb VHCDR2 |
| 48 | Anti-HER2 mAb VHCDR3 |
| 49 | Anti-HER2 mAb VLCDR1 |
| 50 | Anti-HER2 mAb VLCDR2 |

SEQ ID NO: 1: Anti-Trastuzumab mAb heavy chain variable region
SEQ ID NO: 2: Anti-Trastuzumab mAb light chain variable region
SEQ ID NO: 3: Anti-Pertuzumab mAb heavy chain variable region
SEQ ID NO: 4: Anti-Pertuzumab mAb light chain variable region
SEQ ID NO: 5: Anti-HER2 mAb heavy chain variable region 
EVQLVESGGGLVQPGGSLRLSCAASGFNIKDTYIHWVRQAPGKGLEWVARIYPTQ
GYTRYADSVKGRFTISADTSKNTAYLQMNSLRAEDTAVYYCSRWGGEGFYAMDYWG
QGTLVTVSS
SEQ ID NO: 6: Anti-HER2 mAb light chain variable region 
DIQMTQSPSSLSASVGDRVTITCRASQSVQGAVAWYQQKPGKAPKLLIYSASFLYS
GVPSRFSGSRSGTDFTLTISSLQPEDFATYYCQQHSTTPPTFGQGTKVEIK
SEQ ID NO: 7: 4-1BB binding region of anti-4-1BB nanobody AB24 ME 
QVQLVESGGGVVQPGRSLRLSCAASGSTFSIVAMGWYRQAPGKQRELVASIITGDG
DTNYADSVKGRFTISRDNSKNTMYLQMNSLKPEDTAVYYCYARTGYGSSELEGHEYDY
WGQGTQVTVSS
SEQ ID NO: 8: Anti-HER2 mAb VLCDR3
   QQHSTTPPT
SEQ ID NO: 9: Amino acid sequence of human κ light chain constant region (CL)
SEQ ID NO: 10: Amino acid sequence of human IgG1 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSFFLYSKL
TVDKSRWQQGNVFSCSVMHEALHNHYTQKSLSLSPG
SEQ ID NO: 11: Amino acid sequence of human IgG1 (introduced with LALA mutations to reduce Fc function) 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
AAGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSK
LTVDKSRWQQGNVFSCSVMHEALHNHYTQKSLSLSPG
SEQ ID NO: 12: Amino acid sequence of human IgG1 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
 
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKL
TVDKSRWQQGNVFSCSVMHEALHNHYTQKSLSLSPG
SEQ ID NO: 13: CH1/CL preference-mutated human IgG1 Fc amino acid sequence CH SET1 (introduced with Knob mutation) 
ASTKGPSVFPLAPSSKSTSGGTAALGCQVEDYFPEPVTVSWNSGALTSGVHTFPAV
LQSSGLYELSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPCRDELTKNQVSLWCLVKGFYPSDIAVEWESNGQPENNYKTTPPVLDSDGSFFLYSK
LTVDKSRWQQGNVFSCSVMHEALHNHYTQKSLSLSPG
SEQ ID NO: 14: CH1/CL preference-mutated human IgG1 Fc amino acid sequence CH SET2 (introduced with Hole mutation) 
ASTKGPSVFPRAPSSKSTSGGTAALGCLVRDYFPEPVTVSWNSGALTSGVHTFPAV
LQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVCTL
PPSRDELTKNQVSLSCAVKGFYPSDIAVEWESNGQPENNYKTTPPVLDSDGSFFLVSKLT
VDKSRWQQGNVFSCSVMHEALHNHYTQKSLSLSPG
SEQ ID NO: 15: CH1/CL preference-mutated human κ light chain constant region (CL) amino acid sequence CL SET1 
RTVAAPSVFIFPPSDEQLKSGRASVVCLLNNFYPREAKVQWKVDNALQSGNSQESV
TEQDSKDSTYSLSSRLQLSKADYEKHKVYACEVTHQGLSSPVTKSFNRGEC
SEQ ID NO: 16: CH1/CL preference-mutated human κ light chain constant region (CL) amino acid sequence CL SET2 
RTVAAPSVFIFPPSDEELKSGTASVQCLLNNFYPREAKVQWKVDNALQSGNSQESV
TEQDSKDSTYSLSSELTLSKADYEKHKVYACEVTHQGLSSPVTKSFNRGEC
SEQ ID NO: 17: (G4S)4G
   GGGGSGGGGSGGGGSGGGGSG
SEQ ID NO: 18: (G4A)4
   GGGGAGGGGAGGGGAGGGGA
SEQ ID NO: 19: Amino acid sequence of human IgG1 Fc 
THHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPG
SEQ ID NO: 20: Amino acid sequence of human IgG1 Fc (introduced with LALA mutation to reduce Fc function) 
THTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSPG
SEQ ID NO: 21: HER2x4-1BB-1 heavy chain (HER2x4-1BB-1 peptide chain #1) 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDYWG
QGTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGGGGGAGGGGAGGGG
AGGGGAQVQLVESGGGVVQPGRSLRLSCAASGSTFSIVAMGWYRQAPGKQRELVASII
 
TGDGDTNYADSVKGRFTISRDNSKNTMYLQMNSLKPEDTAVYYCYARTGYGSSELEGH
EYDYWGQGTQVTVSS
SEQ ID NO: 22: HER2x4-1BB-1 light chain (HER2x4-1BB-1 peptide chain #2) 
DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
SEQ ID NO: 23: HER2x4-1BB-2 Heavy chain (HER2x4-1BB-2 peptide chain #1) 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDYWG
QGTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPEAAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGGGGGAGGGGAGGGG
AGGGGAQVQLVESGGGVVQPGRSLRLSCAASGSTFSIVAMGWYRQAPGKQRELVASII
TGDGDTNYADSVKGRFTISRDNSKNTMYLQMNSLKPEDTAVYYCYARTGYGSSELEGH
EYDYWGQGTQVTVSS
SEQ ID NO: 24: HER2x4-1BB-2 light chain (HER2x4-1BB-2 peptide chain #2) 
DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
SEQ ID NO: 25: HER2x4-1BB-3 Heavy chain (HER2x4-1BB-3 peptide chain #1) 
EVQLVESGGGLVQPGGSLRLSCAASGFTFTDYTMDWVRQAPGKGLEWVADVNPN
SGGSIYNQRFKGRFTLSVDRSKNTLYLQMNSLRAEDTAVYYCARNLGPSFYFDYWGQG
TLVTVSSASTKGPSVFPLAPSSKSTSGGTAALGCLVKDYFPEPVTVSWNSGALTSGVHTF
 
PAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLY
SKLTVDKSRWQQGNVFSCSVMHEALHNHYTQKSLSLSPGGGGGAGGGGAGGGGAGG
GGAQVQLVESGGGVVQPGRSLRLSCAASGSTFSIVAMGWYRQAPGKQRELVASIITGD
GDTNYADSVKGRFTISRDNSKNTMYLQMNSLKPEDTAVYYCYARTGYGSSELEGHEYD
YWGQGTQVTVSS
SEQ ID NO: 26: HER2x4-1BB-3 light chain (HER2x4-1BB-3 peptide chain #2) 
DIQMTQSPSSLSASVGDRVTITCKASQDVSIGVAWYQQKPGKAPKLLIYSASYRYT
GVPSRFSGSGSGTDFTLTISSLQPEDFATYYCQQYYIYPYTFGQGTKVEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO: 27: HER2xHER2x4-1BB-1 heavy chain 1 (HER2xHER2x4-1BB-1 peptide chain #1) 
EVQLVESGGGLVQPGGSLRLSCAASGFNIKDTYIHWVRQAPGKGLEWVARIYPTQ
GYTRYADSVKGRFTISADTSKNTAYLQMNSLRAEDTAVYYCSRWGGEGFYAMDYWG
QGTLVTVSSASTKGPSVFPLAPSSKSTSGGTAALGCQVEDYFPEPVTVSWNSGALTSGV
HTFPAVLQSSGLYELSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPCRDELTKNQVSLWCLVKGFYPSDIAVEWESNGQPENNYKTTPPVLDSDGS
FFLYSKLTVDKSRWQQGNVFSCSVMHEALHNHYTQKSLSLSPGGGGGAGGGGAGGGG
AGGGGAQVQLVESGGGVVQPGRSLRLSCAASGSTFSIVAMGWYRQAPGKQRELVASII
TGDGDTNYADSVKGRFTISRDNSKNTMYLQMNSLKPEDTAVYYCYARTGYGSSELEGH
EYDYWGQGTQVTVSS
SEQ ID NO: 28: HER2xHER2x4-1BB-1 light chain 1 (HER2xHER2x4-1BB-1 peptide chain #2) 
DIQMTQSPSSLSASVGDRVTITCRASQSVQGAVAWYQQKPGKAPKLLIYSASFLYS
GVPSRFSGSRSGTDFTLTISSLQPEDFATYYCQQHSTTPPTFGQGTKVEIKRTVAAPSVFIF
PPSDEQLKSGRASVVCLLNNFYPREAKVQWKVDNALQSGNSQESVTEQDSKDSTYSLSS
RLQLSKADYEKHKVYACEVTHQGLSSPVTKSFNRGEC
SEQ ID NO: 29: HER2xHER2x4-1BB-1 heavy chain 2 (HER2xHER2x4-1BB-1 peptide chain #3) 
EVQLVESGGGLVQPGGSLRLSCAASGFTFTDYTMDWVRQAPGKGLEWVADVNPN
SGGSIYNQRFKGRFTLSVDRSKNTLYLQMNSLRAEDTAVYYCARNLGPSFYFDYWGQG
TLVTVSSASTKGPSVFPRAPSSKSTSGGTAALGCLVRDYFPEPVTVSWNSGALTSGVHTF
PAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VCTLPPSRDELTKNQVSLSCAVKGFYPSDIAVEWESNGQPENNYKTTPPVLDSDGSFFLV
SKLTVDKSRWQQGNVFSCSVMHEALHNHYTQKSLSLSPGGGGGAGGGGAGGGGAGG
GGAQVQLVESGGGVVQPGRSLRLSCAASGSTFSIVAMGWYRQAPGKQRELVASIITGD
GDTNYADSVKGRFTISRDNSKNTMYLQMNSLKPEDTAVYYCYARTGYGSSELEGHEYD
YWGQGTQVTVSS
SEQ ID NO: 30: HER2xHER2x4-1BB-1 light chain 2 (HER2xHER2x4-1BB-1 peptide chain #4) 
DIQMTQSPSSLSASVGDRVTITCKASQDVSIGVAWYQQKPGKAPKLLIYSASYRYT
GVPSRFSGSGSGTDFTLTISSLQPEDFATYYCQQYYIYPYTFGQGTKVEIKRTVAAPSVFI
FPPSDEELKSGTASVQCLLNNFYPREAKVQWKVDNALQSGNSQESVTEQDSKDSTYSLS
SELTLSKADYEKHKVYACEVTHQGLSSPVTKSFNRGEC
SEQ ID NO: 31: Anti-4-1BB VHH-1 
THTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGGGGGSGGG
GSGGGGSGGGGSGQVQLVESGGGVVQPGRSLRLSCAASGSTFSIVAMGWYRQAPGKQ
 
RELVASIITGDGDTNYADSVKGRFTISRDNSKNTMYLQMNSLKPEDTAVYYCYARTGY
GSSELEGHEYDYWGQGTQVTVSS
SEQ ID NO: 32: Anti-4-1BB VHH-2 
THTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSPGGGGGSGG
GGSGGGGSGGGGSGQVQLVESGGGVVQPGRSLRLSCAASGSTFSIVAMGWYRQAPGK
QRELVASIITGDGDTNYADSVKGRFTISRDNSKNTMYLQMNSLKPEDTAVYYCYARTG
YGSSELEGHEYDYWGQGTQVTVSS
SEQ ID NO: 33: Urelumab heavy chain (Urelumab peptide chain #1) 
QVQLQQWGAGLLKPSETLSLTCAVYGGSFSGYYWSWIRQSPEKGLEWIGEINHGG
YVTYNPSLESRVTISVDTSKNQFSLKLSSVTAADTAVYYCARDYGPGNYDWYFDLWGR
GTLVTVSSASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPCPAP
EFLGGPSVFLFPPKPKDTLMISRTPEVTCVVVDVSQEDPEVQFNWYVDGVEVHNAKTKP
REEQFNSTYRVVSVLTVLHQDWLNGKEYKCKVSNKGLPSSIEKTISKAKGQPREPQVYT
LPPSQEEMTKNQVSLTCLVKGFYPSDIAVEWESNGQPENNYKTTPPVLDSDGSFFLYSRL
TVDKSRWQEGNVFSCSVMHEALHNHYTQKSLSLSLG
SEQ ID NO: 34: Urelumab light chain (Urelumab peptide chain #2) 
EIVLTQSPATLSLSPGERATLSCRASQSVSSYLAWYQQKPGQAPRLLIYDASNRATG
IPARFSGSGSGTDFTLTISSLEPEDFAVYYCQQRSNWPPALTFGGGTKVEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 35: PRS-343 heavy chain (PRS-343 peptide chain #1) 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDYWG
QGTLVTVSSASTKGPSVFPLAPCSRSTSESTAALGCLVKDYFPEPVTVSWNSGALTSGVH
 
TFPAVLQSSGLYSLSSVVTVPSSSLGTKTYTCNVDHKPSNTKVDKRVESKYGPPCPPCPA
PEAAGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPREPQV
YTLPPSQEEMTKNQVSLTCLVKGFYPSDIAVEWESNGQPENNYKTTPPVLDSDGSFFLYS
RLTVDKSRWQEGNVFSCSVMHEALHNHYTQKSLSLSLGKGGGGSGGGGSGGGGSQDS
TSDLIPAPPLSKVPLQQNFQDNQFHGKWYVVGQAGNIRLREDKDPIKMMATIYELKEDK
SYDVTMVKFDDKKCMYDIWTFVPGSQPGEFTLGKIKSFPGHTSSLVRVVSTNYNQHAM
VFFKFVFQNREEFYITLYGRTKELTSELKENFIRFSKSLGLPENHIVFPVPIDQCIDG
SEQ ID NO: 36: PRS-343 light chain (PRS-343 peptide chain #2) 
DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
SEQ ID NO: 37: Human IgG1 amino acid sequence HC-1 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
EPPSRDELTKNQVSLTCLVKGFYPSDIAVEWESNGQPENNYKTTPPVLDSDGSFFLLSVL
TVDKSRWQQGNVFSCSVMHEALHNHYTQKSLSLSPG
SEQ ID NO: 38: Human IgG1 amino acid sequence HC-2 
ASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYT
LPPSRDELTKNQVSLLCLVKGFYPSDIAVEWESNGQPENNYKTTPPVLRSDGSFFLYSKL
TVDKSRWQQGNVFSCSVMHEALHNHYTQKSLSLSPG
SEQ ID NO: 39: HER2xHER2x4-1B-2 heavy chain 1 (HER2xHER2x4-1B-2 Peptide chain #1) 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDYWG
QGTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTEPPSRDELTKNQVSLTCLVKGFYPSDIAVEWESNGQPENNYKTTPPVLDSDGSF
FLLSVLTVDKSRWQQGNVFSCSVMHEALHNHYTQKSLSLSPGGGGGAGGGGAGGGGA
GGGGAQVQLVESGGGVVQPGRSLRLSCAASGSTFSIVAMGWYRQAPGKQRELVASIIT
GDGDTNYADSVKGRFTISRDNSKNTMYLQMNSLKPEDTAVYYCYARTGYGSSELEGHE
YDYWGQGTQVTVSS
SEQ ID NO: 40: HER2xHER2x4-1B-2 light chain 1 (HER2xHER2x4-1BB-2 peptide chain #2) 
DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
SEQ ID NO: 41: HER2xHER2x4-1BB-2 heavy chain 2 (HER2xHER2x4-1BB-2 peptide chain #3) 
EVQLVESGGGLVQPGGSLRLSCAASGFTFTDYTMDWVRQAPGKGLEWVADVNPN
SGGSIYNQRFKGRFTLSVDRSKNTLYLQMNSLRAEDTAVYYCARNLGPSFYFDYWGQG
TLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQ
VYTLPPSRDELTKNQVSLLCLVKGFYPSDIAVEWESNGQPENNYKTTPPVLRSDGSFFLY
SKLTVDKSRWQQGNVFSCSVMHEALHNHYTQKSLSLSPGGGGGAGGGGAGGGGAG
GGGAQVQLVESGGGVVQPGRSLRLSCAASGSTFSIVAMGWYRQAPGKQRELVASIITG
 
DGDTNYADSVKGRFTISRDNSKNTMYLQMNSLKPEDTAVYYCYARTGYGSSELEGHEY
DYWGQGTQVTVSS
SEQ ID NO: 42: HER2x HER2x4-1BB-2 light chain 2 (HER2x HER2x4-1BB-2 peptide chain #4) 
DIQMTQSPSSLSASVGDRVTITCKASQDVSIGVAWYQQKPGKAPKLLIYSASYRYT
GVPSRFSGSGSGTDFTLTISSLQPEDFATYYCQQYYIYPYTFGQGTKVEIKRTVAAPSVFI
FPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC
SEQ ID NO: 43: CDR1 of anti-4-1BB nanobody AB24 ME
   GSTFSIVA
SEQ ID NO: 44: CDR2 of anti-4-1BB nanobody AB24 ME
   IITGDGDT
SEQ ID NO: 45: CDR3 of anti-4-1BB nanobody AB24 ME
   YARTGYGSSELEGHEYDY
SEQ ID NO: 46: Anti-HER2 mAb VHCDR1
   GFNIKDTY
SEQ ID NO: 47: Anti-HER2 mAb VHCDR2
   IYPTQGYT
SEQ ID NO: 48: Anti-HER2 mAb VHCDR3
   SRWGGEGFYAMDY
SEQ ID NO: 49: Anti-HER2 mAb VLCDR1
   QSVQGA
SEQ ID NO: 50: Anti-HER2 mAb VLCDR2
   SAS

### Example 1. Cloning and expression of multi-specific antibodies

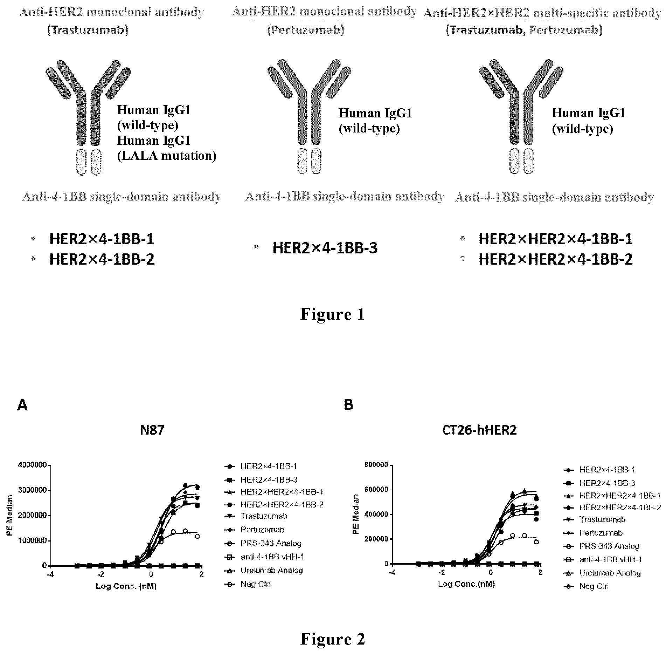

In this example, three HER2x4-1BB bispecific antibodies, two anti-HER2xHER2x4-1BB trispecific antibodies and two 4-1BB monoclonal antibodies were constructed, respectively:
HER2x4-1BB-1: It consisted of 2 polypeptide chains, and its structural schematic diagram was shown in Figure 1. Peptide chain #1 had an amino acid sequence as set forth in SEQ ID NO: 21, which contained the amino acid sequence (SEQ ID NO: 1) of the heavy chain variable region of the anti-HER2 monoclonal antibody Trastuzumab (patent application number: WO1992022653A1) and the human IgG1 amino acid sequence (SEQ ID NO: 10). The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #2 had an amino acid sequence as set forth in SEQ ID NO: 22, which contained the amino acid sequence (SEQ ID NO: 2) of the light chain variable region of the anti-HER2 monoclonal antibody Trastuzumab (patent application number: WO1992022653A1), and the amino acid sequence (SEQ ID NO: 9) of the human κ light chain constant region (CL) at the C-terminal of the VL amino acid sequence.

HER2x4-1BB-2: It consisted of 2 polypeptide chains, and its structural schematic diagram was shown in Figure 1. Peptide chain #1 had an amino acid sequence as set forth in SEQ ID NO: 23, which contained the heavy chain variable region amino acid sequence (SEQ ID NO: 1) of the anti-HER2 monoclonal antibody Trastuzumab (patent application number: WO1992022653A1) and the human IgG1 amino acid sequence (introduced with LALA mutation to reduce Fc function, SEQ ID NO: 11). The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #2 had an amino acid sequence as set forth in SEQ ID NO: 24, which contained the amino acid sequence (SEQ ID NO: 2) of the light chain variable region of the anti-HER2 monoclonal antibody Trastuzumab (patent application number: WO1992022653A1), and the amino acid sequence (SEQ ID NO: 9) of the human κ light chain constant region (CL) at the C-terminal of the VL amino acid sequence.

HER2x4-1BB-3: It consisted of 2 polypeptide chains, and its structural schematic diagram was shown in Figure 1. Peptide chain #1 had an amino acid sequence as set forth in SEQ ID NO: 25, which contained the amino acid sequence (SEQ ID NO: 3) of the heavy chain variable region of the anti-HER2 monoclonal antibody Pertuzumab (patent application number: US7449184) and the human IgG1 amino acid sequence (SEQ ID NO: 12). The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #2 had an amino acid sequence as set forth in SEQ ID NO: 26, which contained the light chain variable region amino acid sequence (SEQ ID NO: 4) of the anti-HER2 monoclonal antibody Pertuzumab (patent application number: US7449184), and the human κ light chain constant region (CL) amino acid sequence (SEQ ID NO: 9) at the C-terminal of the VL amino acid sequence.

HER2xHER2x4-1BB-1: It consisted of 4 polypeptide chains, and its structural schematic diagram was shown in Figure 1. Peptide chain #1 had the amino acid sequence as set forth in SEQ ID NO: 27, which contained the amino acid sequence (SEQ ID NO: 5) of the heavy chain variable region of the anti-HER2 monoclonal antibody and the human IgG1 amino acid sequence (introduced with CH3 Knob mutation and CH1/CL preference mutation) CH SET1 (patent application number: WO2021067404A2, SEQ ID NO: 13). The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #2 had an amino acid sequence as set forth in SEQ ID NO: 28, which contained the amino acid sequence (SEQ ID NO: 6) of the light chain variable region of the anti-HER2 monoclonal antibody, and the human κ light chain constant region (CL) amino acid sequence (introduced with CH1/CL preference mutation) CL SET1 (patent application number: WO2021067404A2, SEQ ID NO: 15) at the C-terminal of the VL amino acid sequence. Peptide chain #3 had an amino acid sequence as set forth in SEQ ID NO: 29, which contained the amino acid sequence (SEQ ID NO: 3) of the heavy chain variable region of the anti-HER2 monoclonal antibody Pertuzumab (patent application number: US7449184) and the human IgG1 amino acid sequence (introduced with CH3 Hole mutation and CH1/CL preference mutation) CH SET2 (patent application number: WO2021067404A2, SEQ ID NO: 14). The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #4 had an amino acid sequence as set forth in SEQ ID NO: 30, which contained the light chain variable region amino acid sequence (SEQ ID NO: 4) of the anti-HER2 monoclonal antibody Pertuzumab (patent application number: US7449184), and the human κ light chain constant region (CL) amino acid sequence (introduced with CH1/CL preference mutation) CL SET2 (patent application number: WO2021067404A2, SEQ ID NO: 16) at the C-terminal of the VL amino acid sequence.

HER2xHER2x4-1BB-2: It consisted of 4 polypeptide chains, and its structural schematic diagram was shown in Figure 1. Peptide chain #1 was synthesized according to the method described in the patent (patent application number: 201611016435.0), and had an amino acid sequence as set forth in SEQ ID NO: 39, which contained the heavy chain variable region amino acid sequence (SEQ ID NO: 1) of the anti-HER2 monoclonal antibody Trastuzumab (patent application number: WO1992022653A1) and the human IgG1 amino acid sequence HC-1 (SEQ ID NO: 37, patent application number: PCT/CN2017/111310) that could spontaneously form heterodimer. The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #2 had an amino acid sequence as set forth in SEQ ID NO: 40, which contained the light chain variable region amino acid sequence (SEQ ID NO: 2) of the anti-HER2 monoclonal antibody Trastuzumab (patent application number: WO1992022653A1), and the human κ light chain constant region (CL) amino acid sequence (SEQ ID NO: 9) at the C-terminal of the VL amino acid sequence. Peptide chain #3, according to the synthetic method described in the patent (patent application number: 201611016435.0), had an amino acid sequence as set forth in SEQ ID NO: 41, which contained the heavy chain variable region amino acid sequence (SEQ ID NO: 3) of the anti-HER2 monoclonal antibody Pertuzumab (patent application number: US7449184), and the human IgG1 amino acid sequence HC-2 (SEQ ID NO: 38, patent application number: PCT/CN2017/111310) that could spontaneously form heterodimer. The N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). Peptide chain #4 had an amino acid sequence as set forth in SEQ ID NO: 42, which contained the light chain variable region amino acid sequence (SEQ ID NO: 4) of the anti-HER2 monoclonal antibody Pertuzumab (patent application number: US7449184), and the human κ light chain constant region (CL) amino acid sequence (SEQ ID NO: 9) at the C-terminal of the VL amino acid sequence.

Anti-4-1BB-VHH-1: It consisted of 2 polypeptide chains and had an amino acid sequence as set forth in SEQ ID NO: 31, which contained the human IgG1 Fc amino acid sequence (SEQ ID NO: 19), and the N-terminal of the CD137 binding region amino acid sequence (SEQ ID NO: 7) of the anti-CD137 single-domain antibody AB24 ME was linked to the C-terminal of Fc through a flexible peptide of 21 amino acid residues (G4S)4G (SEQ ID NO: 17).

Anti-4-1BB-VHH-2: It consisted of 2 polypeptide chains and had and amino acid sequence as set forth in SEQ ID NO: 32, which contained the human IgG1 Fc amino acid sequence (introduced with LALA mutation to reduce Fc function, SEQ ID NO: 20), and the N-terminal of the CD137 binding region amino acid sequence (SEQ ID NO: 7) of the anti-CD137 single-domain antibody AB24 ME was linked to the C-terminal of Fc through a flexible peptide of 21 amino acid residues (G4S)4G (SEQ ID NO: 17).

In this example, positive control molecules Urelumab and PRS343 were constructed:
Urelumab: The control molecule Urelumab (patent application number: WO2004010947) consisted of 2 polypeptide chains, in which Peptide chain #1 had an amino acid sequence as set forth in SEQ ID NO: 33, and Peptide chain #2 had an amino acid sequence as set forth in SEQ ID NO: 34.

PRS-343: The control molecule PRS-343 (patent application number: WO2016177802A1) consisted of 2 polypeptide chains, in which Peptide chain #1 had an amino acid sequence as set forth in SEQ ID NO: 35, and Peptide chain #2 had an amino acid sequence as set forth in SEQ ID NO: 36.

### Example 2. Multi-specific antibody binding to human HER2

In this experiment, N87 cells (self-expressing HER2) and CT26-hHER2 (overexpressing human HER2) cells that had been expanded and cultured were digested with 0.25% EDTA trypsin, washed once with culture medium. The cell density was adjusted to 2×10⁶ cells/ml. The resulting suspension was added to a 96-well flow plate at 100 µl/well, and centrifuged for later use. Gradiently diluted antibodies were added to the above 96-well flow plate with cells at 100 µl/well and incubated at 4°C for 60 min. After two washes with PBS, Goat anti-human IgG-Fc (PE) (Abcam, ab98596) diluted 1000 times with 2% BSA solution was added at 100 µl/well, and incubated at 4°C for 60 min. After two washes with PBS, the cells were finally resuspended in PBS at 100 µl/well, and detected by a CytoFlex (Beckman) flow cytometer, and the corresponding mean fluorescence intensity (MFI) was calculated.

The experimental results were shown in Figure 2 and Tables 1-2. All of the HER2xHER2x4-1BB trispecific antibodies and HER2x4-1BB bispecific antibodies of the present invention were capable of binding to HER2 expressed on human gastric cancer cells N87 (Figure 2A) and human HER2-overexpressing CT26 cells (Figure 2B), and the binding activity was comparable to that of HER2 monoclonal antibodies (Trastuzumab or Pertuzumab).

**Table 1: Binding of multi-specific antibodies to HER2 overexpressed on human gastric cancer cells N87**

| Antibody | EC₅₀ (nM) |
|---|---|
| HER2 × 4-1BB-1 | 1.876 |
| HER2×4-1BB-3 | 2.769 |
| HER2×HER2×4-1BB-1 | 2.650 |
| HER2×HER2×4-1BB-2 | 2.877 |
| Trastuzumab | 1.441 |
| Pertuzumab | 1.737 |
| PRS-343 Analog | 1.253 |
| anti-4-1BB vHH-1 | N/A |
| Urelumab Analog | N/A |
| Neg Ctrl | N/A |

**Table 2: Binding of multi-specific antibodies to human HER2 overexpressed on CT26**

| Antibody | EC₅₀ (nM) |
|---|---|
| HER2×4-1BB-1 | 1.170 |
| HER2×4-1BB-3 | 1.755 |
| HER2×HER2×4-1BB-1 | 1.823 |
| HER2×HER2×4-1BB-2 | 1.812 |
| Trastuzumab | 1.084 |
| Pertuzumab | 1.171 |
| PRS-343 Analog | 1.080 |
| anti-4-1BB vHH-1 | N/A |
| Urelumab Analog | N/A |
| Neg Ctrl | N/A |

### Example 3. Binding of multi-specific antibodies to human 4-1BB

In this experiment, CHOS-h4-1BB (overexpressing human 4-1BB) cells that had been expanded and cultured were adjusted to reach a cell density of 2×10⁶ cells/ml, added to a 96-well flow plate at 100 µl/well, and centrifuged for later use. Gradiently diluted antibodies were added to the above 96-well flow plate with cells at 100 µl/well and incubated at 4°C for 60 min. After two washes with PBS, Goat anti-human IgG-Fc (PE) (Abcam, ab98596) diluted 1000 times with 2% BSA solution was added at 100 µl/well, and incubated at 4°C for 60 min. After two washes with PBS, the cells were finally resuspended in PBS at 100 µl/well, and detected by a CytoFlex (Beckman) flow cytometer, and the corresponding MFI was calculated.

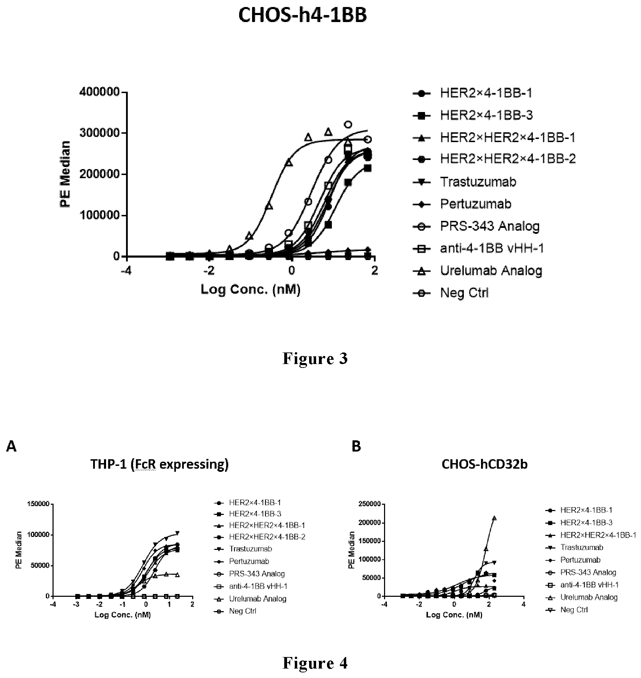

The experimental results were shown in Figure 3 and Table 3. All of the HER2xHER2x4-1BB trispecific antibodies and HER2x4-1BB bispecific antibodies of the present invention were capable of binding to CHOS-h4-1BB cells, and the binding activity was comparable to that of 4-1BB monoclonal antibody (anti-4-1BB VHH), which was weaker than that of the control antibodies Urelumab and PRS-343.

**Table 3: Binding of multi-specific antibodies to human 4-1BB overexpressed on CHOS cells**

| Antibody | EC₅₀ (nM) |
|---|---|
| HER2 × 4-1BB-1 | 7.853 |
| HER2 × 4-1BB-3 | 10.900 |
| HER2 × HER2 × 4-1BB-1 | 6.990 |
| HER2 × HER2 × 4-1BB-2 | 6.003 |
| Trastuzumab | N/A |
| Pertuzumab | N/A |
| PRS-343 Analog | 2.960 |
| anti-4-1BB vHH-1 | 4.605 |
| Urelumab Analog | 0.316 |
| Neg Ctrl | N/A |

### Example 4. Binding of multi-specific antibodies to human FcR

In this experiment, THP-1 (self-expressing FcRs) and CHOS-hCD32b (overexpressing human FcγRIIb) cells that had been expanded and cultured were adjusted to reach a cell density of 2×10⁶ cells/ml, added to a 96-well flow plate at 100 µl/well, and centrifuged for later use. Gradiently diluted antibodies were added to the above 96-well flow plate with cells at 100 µl/well and incubated at 4°C for 60 min. After two washes with PBS, Goat anti-human IgG-Fc (PE) (Abcam, ab98596) diluted 1000 times with 2% BSA solution was added at 100 µl/well, and incubated at 4°C for 60 min. After two washes with PBS, the cells were finally resuspended in PBS at 100 µl/well, and detected by a CytoFlex (Beckman) flow cytometer, and the corresponding mean fluorescence intensity (MFI) was calculated.

The experimental results were shown in Figure 4 and Tables 4-5. All of the HER2xHER2x4-1BB trispecific antibodies and HER2x4-1BB bispecific antibodies retaining Fc effector function of the present invention were capable of binding to FcγRIIb expressed on THP-1 (self-expressing FcRs, Figure 4A) and CHOS-hCD32b (overexpressing human FcR, Figure 4B) cells. The Fc of the HER2xHER2x4-1BB trispecific antibody adopted Knob in Hole mutation, so its binding activity with FcR was slightly weaker than that of the HER2x4-1BB bispecific antibody and HER2 monoclonal antibody adopting wild-type human IgG1 Fc. The Fc of Urelumab and PRS-343 adopted wild-type human IgG4 Fc or human IgG4 Fc carrying FALA mutation, respectively, so their affinity with FcR was weaker than that of multi-specific antibodies retaining Fc effector function.

**Table 4: Binding of multi-specific antibodies to FcR (THP-1)**

| Antibody | EC₅₀ (nM) |
|---|---|
| HER2 × 4-1BB-1 | 1.224 |
| HER2 × 4-1BB-3 | 1.053 |
| HER2 × HER2 × 4-1BB-1 | 1.224 |
| HER2 × HER2 × 4-1BB-2 | 2.274 |
| Trastuzumab | 0.731 |
| Pertuzumab | 0.716 |
| PRS-343 Analog | N/A |
| anti-4-1BB vHH-1 | N/A |
| Urelumab Analog | 0.462 |
| Neg Ctrl | N/A |

**Table 5: Binding of multi-specific antibodies to FcR (CHOS-hCD32b)**

| Antibody | EC₅₀ (nM) |
|---|---|
| HER2×4-1BB-1 | 52.600 |
| HER2 × 4-1BB-3 | 3.416 |
| HER2×HER2×4-1BB-1 | 1.064 |
| Trastuzumab | 14.120 |
| Pertuzumab | 1.355 |
| PRS-343 Analog | N/A |
| anti-4-1BB vHH-1 | N/A |
| Urelumab Analog | 65.240 |
| Neg Ctrl | N/A |

### Example 5. Inhibiting proliferation of HER2 signal-dependent cells by multi-specific antibodies

In this experiment, N87 and SK-OV-3 (self-expressing HER2) cells that had been expanded and cultured were digested with 0.25% EDTA trypsin, washed once with culture medium. The cell density was adjusted to 5×10⁴ cells/ml. The resulting suspension was added to a 96-well plate at 80µl/well for later use. Gradiently diluted antibodies were added to the above 96-well plate with cells at 80 µl/well and incubated in a cell culture incubator for 3-5 days. Finally, a CellTiter-Glo^{®} Luminescent Cell Viability Assay (Promega, G7572) kit was used for signal generation, and the chemiluminescent signals were collected using a microplate reader.

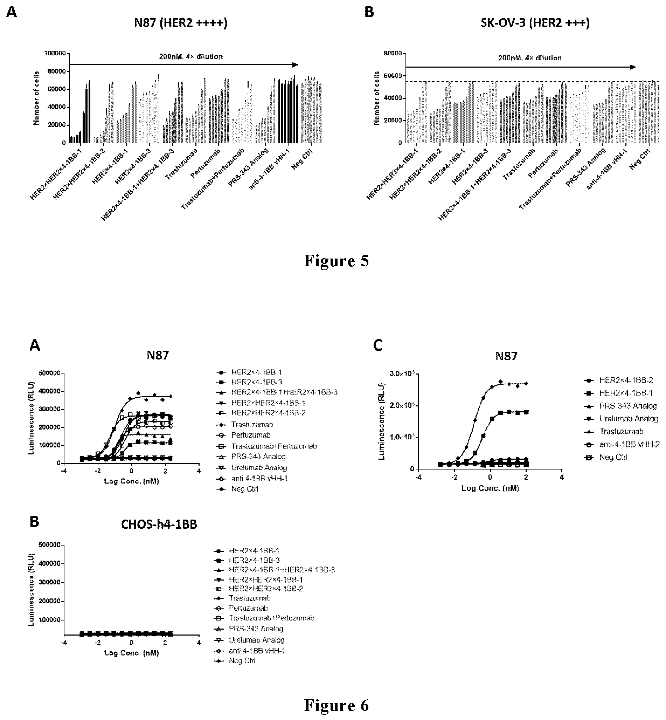

As shown in Figures 5A and 5B, the multi-specific antibodies were capable of significantly inhibiting the proliferation of N87 or SK-OV-3 cells, wherein the HER2xHER2x4-1BB trispecific antibody had a significantly stronger inhibitory effect on cell proliferation than that of the HER2-4-1BB bispecific antibody or HER2 monoclonal antibody. Furthermore, the inhibitory effect of HER2 antibody on tumor cells positively correlated with HER2 expression levels on the cells. The higher the HER2 expression level, the more pronounced the antibody's inhibitory effect on cell proliferation became.

### Example 6. Induction of ADCC effect (reporter gene) by multi-specific antibodies

In this experiment, N87 (self-expressing HER2) cells and CHOS-h4-1BB (overexpressing human 4-1BB) cells that had been expanded and cultured were mixed with 1.2×10⁵/well of NFAT Luciferase/Jurkat CD16a (overexpressing CD16a and NFAT-Luc) effector cells at 3×10⁴/well, and inoculated into a 96-well cell culture white bottom plate. Then, gradiently diluted multi-specific antibodies were added to the 96-well plate and mixed, and incubated in a cell culture incubator for 6 hours. A Bio-Glo luciferase assay system (Promega, G7940) kit was for signal generation, and the chemiluminescent signals were collected using an ELISA instrument.

The experimental results were shown in Figures 6A and 6C and Tables 6 to 7. The multi-specific antibodies that retained the Fc effector function could mediate ADCC effect through HER2 expressed on N87 cells, thereby activating the CD16a-NFAT signaling pathway on Jurkat cells. In contrast, neither the HER2x4-1BB-2 bispecific antibody with LALA mutation to abrogate Fc effector function nor the control molecule PRS-343 based on IgG4 Fc with FALA mutation demonstrated ADCC effect. In addition, when CHOS-h4-1BB cells were used as target cells, as shown in Figure 6B, none of the multi-specific antibodies exhibited detectable ADCC effect against 4-1BB cells.

**Table 6: ADCC effect induced by multi-specific antibodies (corresponding to Figure 6A)**

| Antibody | EC₅₀ (nM) |
|---|---|
| HER2×4-1BB-1 | 0.232 |
| HER2×4-1BB-3 | 0.367 |
| HER2×4-1BB-1+HER2×4-1BB-3 | 0.114 |
| HER2×HER2×4-1BB-1 | 0.313 |
| HER2×HER2×4-1BB-2 | 0.391 |
| Trastuzumab | 0.097 |
| Pertuzumab | 0.185 |
| Trastuzumab+Pertuzumab | 0.051 |
| PRS-343 Analog | N/A |
| Urelumab Analog | N/A |
| anti 4-1BB vHH-1 | N/A |
| Neg Ctrl | N/A |

**Table 7: ADCC effect induced by multi-specific antibodies (corresponding to Figure 6C)**

| Antibody | EC₅₀ (nM) |
|---|---|
| HER2×4-1BB-2 | N/A |
| HER2×4-1BB-1 | 0.360 |
| PRS-343 Analog | N/A |
| Urelumab Analog | N/A |
| Trastuzumab | 0.121 |
| anti 4-1BB vHH-2 | N/A |
| Neg Ctrl | N/A |

### Example 7. Induction of primary T cells to release cytokines (mediated by HER2+ cells) by multi-specific antibodies

The target cells N87 (self-expressing HER2) that had been expanded and cultured were digested with 0.25% EDTA trypsin, and inoculated into a 96-well culture plate at 1×10⁴ cells/well (the culture plate was coated with 2µg/ml anti-CD3 antibody at 100µl/well one day in advance). PBMCs recovered one day in advance were collected, and the T cells in PBMCs were separated using a T cell separation kit (Stemcell, 17951) and added to the target cell wells at 5×10⁴/well. Then, the gradiently diluted multi-specific antibodies were added to the cell wells and incubated for 48 hours, and then the supernatant was collected. The levels of IL-2 and IFNγ in the supernatant were detected using a human IL-2 ELISA kit (Invitrogen, 88-7025-77) and a human IFNγ ELISA kit (Invitrogen, 88-7316-77).

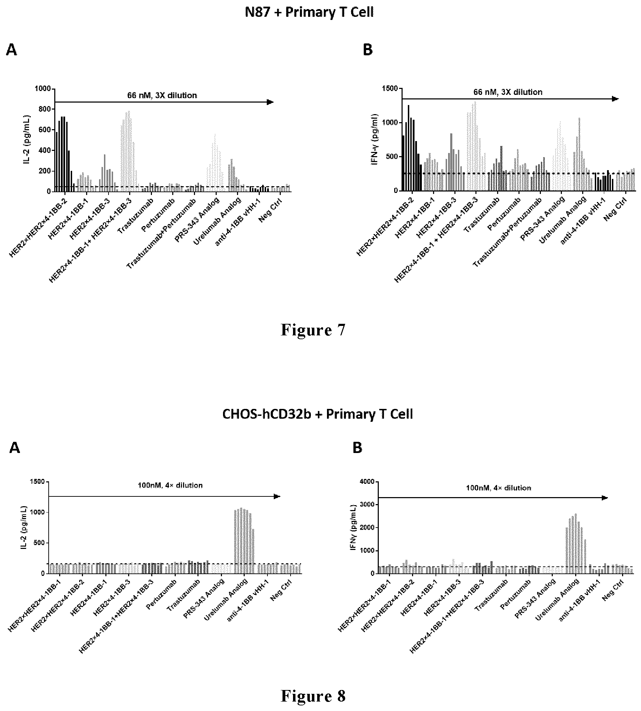

The experimental results were shown in Figure 7. All of the multi-specific antibodies of the present invention could induce primary T cells to release IL-2 (Figure 7A) or IFN-γ (Figure 7B) in the presence of N87 cells, and exhibited dose-dependency. Furthermore, the HER2xHER2x4-1BB trispecific antibody demonstrated significantly stronger cytokine release induction ability compared to the HER2x4-1BB bispecific antibody, as well as the control antibodies PRS-343 and Urelumab.

### Example 8. Induction of primary T cells to release cytokines (FcR+ cell-mediated) by multi-specific antibodies

The target cells CHOS-hCD32b (overexpressing human FcγRIIb) that had been expanded and cultured were centrifuged and counted, and inoculated into a 96-well culture plate at 1×10⁴ cells/well (the culture plate was coated with 2µg/ml anti-CD3 antibody at 100µl/well one day in advance). PBMCs recovered one day in advance were collected, the T cells in PBMCs were separated using a T cell separation kit (Stemcell, 17951) and added to the target cell wells at 5×10⁴ cells/well. Then, the gradiently diluted multi-specific antibodies were added to the cell wells and incubated for 48 hours, and then the supernatant was collected. The levels of IL-2 and IFNγ in the supernatant were detected using a human IL-2 ELISA kit (Invitrogen, 88-7025-77) and a human IFNγ ELISA kit (Invitrogen, 88-7316-77).

The experimental results were shown in Figure 8. None of the multi-specific antibodies of the present invention were able to induce the release of IL2 (Figure 8A) or IFN-γ (Figure 8B) by primary T cells in the presence of CHOS-hCD32b cells. In contrast, the positive control antibody, Urelumab, successfully induced T cell activation and the release of proinflammatory factors through FcR engagement. In addition, the negative control molecule, PRS-343, did not bind to CHOS-hCD32b cells, thereby failing to induce cytokines release from primary T cells through FcR interaction.

### Example 9. Screening of cell lines

In this example, CH1/CL preference mutation (patent number: WO2021/067404A2) and Knob in hole technology were used to construct the heavy chain variable regions of anti-HER2 antibody and Pertuzumab antibody into CH1-mutated CH SET1 (SEQ ID NO: 13) and CH SET2 (SEQ ID NO: 14), respectively, and the N-terminal of the CD137 binding region amino acid sequence (SEQ ID NO: 7) of the anti-CD137 single-domain antibody AB24 ME was linked to the C-terminal of Fc through a flexible peptide of 20 amino acid residues (G4A)4 (SEQ ID NO: 18). The light chain variable regions of anti-HER2 antibody and Pertuzumab antibody were constructed into CL SET1 (SEQ ID NO: 15) and CL SET2 (SEQ ID NO: 16) on the CL mutant light chain constant region, respectively. A 1+1 asymmetric anti-HER2xHER2x4-1BB-1 trispecific antibody cell line was constructed.

### Cell line construction

The vector pCHO2.0-GS-Puro-H1-L1, carrying the heavy chain gene (SEQ ID NO: 27) and light chain gene (SEQ ID NO: 28) of anti-HER2 antibody and anti-4-1BB antibody, and the vector pCHO2.0-GS-Puro-H2-L2, carrying the heavy chain gene (SEQ ID NO: 29) and light chain gene (SEQ ID NO: 30) of anti-Pertuzuamb anti-4-1BB antibody were co-transfected into the host cell CHOS-ADP via electroporation. Puromycin and MSX screening pressures were applied to perform pressure screening on the transfected cells, resulting in the selection of a high-yield minipool. Subsequently, a round of limiting dilution was conducted followed by monoclonal identification to isolate a high-yield stable cell line.

### Cell culture

Dynamis AGT Medium was used as the base medium, the cells were inoculated at a density of (1.0±0.2) ×10⁶ cells/ml, and subjected to fed-batch culture. On the 3^{rd}, 5^{th}, 7^{th}, 9^{th}, and 11^{th} days, Cell boost 7a powder supplement was added at 5.0±0.5% (w/w) of the initial culture weigh, and Cell boost 7b powder supplement was added at 0.5±0.05% (w/w) of the initial culture weight. The dissolved oxygen was set at 40%, with an initial culture temperature of 36.5°C that was reduced to 33.0°C on the 4^{th} day. Based on glucose concentration measurements, 300 g/kg of glucose concentrate was added daily to reach a glucose concentration of 6.0 g/L in the cell culture, except on the day of harvest. The culture was terminated on the 14^{th} day or when cell viability dropped below 80%. Throughout the culture process, cell density and viability were monitored using a Vicell analyzer (Beckman). Starting from the 7^{th} day, antibody production was measured daily using a Cedex analyzer (Roche).

### Quality assessment of stable cell line product

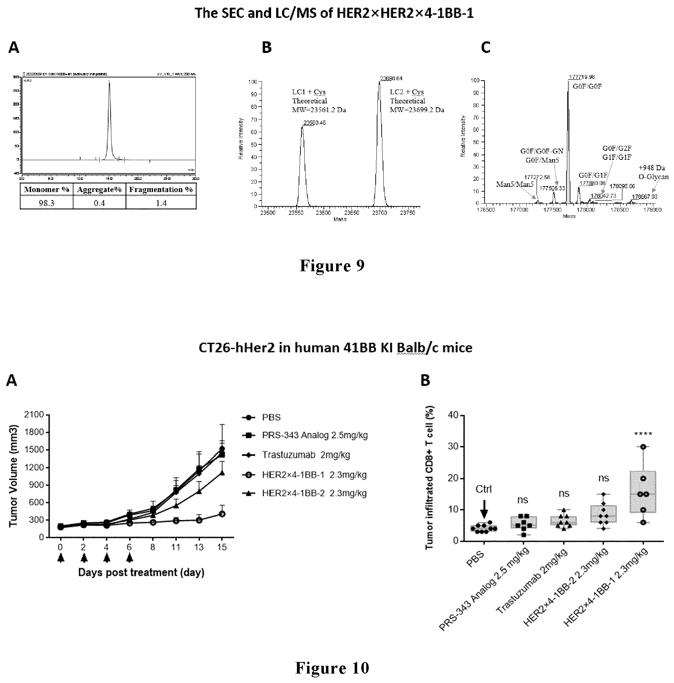

A one-step affinity purification method, identical to that used for the one-arm antibody in Example 1, was employed. HPLC was used to obtain the purity of protein. The HPLC method was as follows: mobile phase: 150 mM Na₂HPO₄•12H₂O, pH7.0. Chromatographic conditions: detection wavelength: 280 nm, column temperature: 25°C, flow rate: 0.5 ml/min, detection time: 30 min, TSKgel G3000SWXL column. The results of SEC were shown in Figure 9A, and the purity of the bispecific antibody obtained by one-step affinity purification was 98.3%.

The pairing of heavy and light chains of protein was detected by high-performance liquid chromatography-mass spectrometry, using a liquid phase system Vanquish UHPLC (Thermo), a mass spectrometer Q Exactive (Thermo), and a chromatographic column Waters ACQUITY UPLC BEH C4, 2.1 mm×100 mm. 50 µg of sample was taken, diluted with ultrapure water to 25 µl, and centrifuged, then 20 µl of sample was taken and transferred into a sample vial, 5 µl was injected, and LC-MS was used to analyze the intact molecular weight. The chromatographic conditions were: column temperature: 80°C; UV detection wavelength: 280 nm; flow rate: 0.3 mL/min; mobile phase A: water containing 0.1% formic acid; mobile phase B: acetonitrile containing 0.1% formic acid. Mass spectrometry parameters were: ESI ion source: ion transfer tube temperature: 320°C, voltage: 3.8 kV, gas flow rate: 36 L/min; mode: positive ion Full MS; resolution: 17500; scanning range: 600-4000 m/z. The results were shown in Figures 9B and 9C. The correct chain pairing ratio of the purified trispecific antibody HER2xHER2x4-1BB-1 exceeded 98%.

### Example 10. Study on tumor inhibitory activity of the multi-specific antibodies

In this experiment, the concept verification of the antitumor activity of the multi-specific antibodies of the present invention were performed in a tumor model using h-4-1BB KI BALB/c mice inoculated subcutaneously with CT26-h-HER2.

First, a tumor-bearing mouse model was established through subcutaneous inoculation of CT26-h-HER2 cells. Once the tumor grew to a volume of about 180 mm³, the mice were randomized into groups and treated by administration via intraperitoneal injection of, G1: PBS, G2: 2.5 mg/kg PRS-343, G3: 2 mg/kg Trastuzumab, G4: 2.3 mg/kg HER2x4-1BB-1, and G5: 2.3 mg/kg HER2x4-1BB-2 (all groups were treated with an equivalent molar dose). The changes in tumor volume and body weight of mice in each group were monitored every 2-3 days/time for a duration of 2-3 weeks. The dosage and administration schedule were shown in Table 8. After the experiment, the tumors of the mice were taken for immunohistochemical detection to compare the infiltration of CD8+T cells in the tumors of different groups.

The experimental results were shown in Figure 10A. The bispecific antibody HER2x4-1BB-1 that retained the Fc effector function exhibited significantly better anti-tumor activity compared to the HER2x4-1BB-2 bispecific antibody with LALA mutation to abrogate Fc effector function (TGI: 73.2% vs 26.6%), as well as the control antibody PRS-343 (TGI: 6.5%) and the HER2 monoclonal antibody Trastuzumab (TGI: 4.2%). Similarly, the results of immunohistochemical detection (Figure 10B) also demonstrated significantly stronger CD8+T cell infiltration in the tumors of mice treated with the HER2x4-1BB-1 bispecific antibody than that in the mice treated with the control antibody PRS-343, Trastuzumab and HER2x4-1BB-2 bispecific antibody.

**Table 8: Dosage regimen for the study on tumor inhibitory activity of the multi-specific antibodies**

| Group | Dosage | Number of dosages × dosage frequency |
|---|---|---|
| G1: Negative control (PBS) | N/A | Q2d × 4 |
| G2: PRS-343 | 2.5 mg/kg | Q2d × 4 |
| G3: Trastuzumab | 2mg/kg | Q2d × 4 |
| G4: HER2x4-1BB-1 | 2.3 mg/kg | Q2d × 4 |
| G5: HER2x4-1BB-2 | 2.3 mg/kg | Q2d × 4 |

### Example 11. Study on tumor inhibition activity of the multi-specific antibodies

In this experiment, the antitumor activity of the multi-specific antibodies of the present invention was evaluated in a tumor model using h-4-1BB KI BALB/c mice that was inoculated subcutaneously with CT26-h-HER2.

First, a tumor-bearing mouse model was established through subcutaneous inoculation of h-HER2 CT26 cells. Once the tumor grew to a volume of about 180 mm³, the mice were randomized into groups and treated by administration via intraperitoneal injection of: G1: PBS, G2: 3mg/kg Trastuzumab combined with 3mg/kg Pertuzumab, G3: 3.6mg/kg PRS-343, G4: 3mg/kg Enhertu (DS8201), G5: 3.6mg/kg HER2x4-1BB-1, G6: 3.6mg/kg HER2x4-1BB-3, G7: 1.8mg/kg HER2x4-1BB-1 combined with 1.8mg/kg HER2x4-1BB-3, G8: 3.6mg/kg HER2xHER2x4-1BB-2 (all groups were treated with an equivalent molar dose). The changes in tumor volume and body weight of mice in each group were monitored every 2-3 days/time for a duration of 2-3 weeks. The dosage and administration schedule were shown in Table 9.

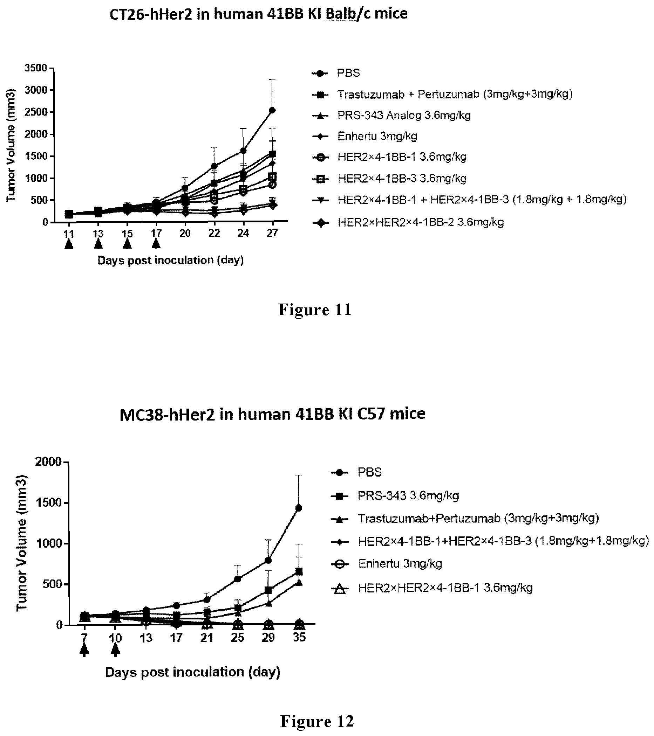

The experimental results were shown in Figure 11. The anti-tumor activity of the HER2xHER2x4-1BB trispecific antibody molecule (TGI: 85.7%) was significantly better than that of the equimolar control antibody PRS-343 (TGI: 37.7%), Enhertu (TGI: 47.7%), and the combination of HER2 monoclonal antibodies Trastuzumab and Pertuzumab (TGI: 39.5%). Furthermore, the HER2xHER2x4-1BB trispecific antibody exhibited significantly stronger anti-tumor activity compared with the HER2x4-1BB bispecific antibodies HER2x4-1BB-1 (TGI: 66.9%) and HER2x4-1BB-3 (TGI: 59.7%), with comparable efficacy to the group treated with the combination of HER2x4-1BB bispecific antibodies (HER2x4-1BB-1+HER2x4-1BB-3, TGI: 83.7%).

**Table 9: Dosage regimen for the study on tumor inhibitory activity of the multi-specific antibodies**

| Group | Dosage | Number of dosages × dosage frequency |
|---|---|---|
| G1: Negative control (PBS) | N/A | Q2d × 4 |
| G2: Trastuzumab+Pertuzumab | 3+3 mg/kg | Q2d × 4 |
| G3: PRS-343 | 3.6mg/kg | Q2d × 4 |
| G4: Enhertu | 3 mg/kg | Q2d × 4 |
| G5: HER2x4-1BB-1 | 3.6 mg/kg | Q2d × 4 |
| G6: HER2x4-1BB-3 | 3.6 mg/kg | Q2d × 4 |
| G7: HER2x4-1BB-1+ HER2x4-1BB-3 | 1.8+1.8mg/kg | Q2d × 4 |
| G8: HER2x HER2x4-1BB-2 | 3.6 mg/kg | Q2d × 4 |

### Example 12. Study on tumor inhibitory activity of the multi-specific antibodies

In this experiment, the antitumor activity of the multi-specific antibodies of the present invention was evaluated in a tumor model using h-4-1BB KI C57 mice that were subcutaneously inoculated with MC38-h-HER2.

First, a tumor-bearing mouse model was established through subcutaneous inoculation of MC38-h-HER2 cells. Once the tumor grew to a volume of about 100 mm³, the mice were randomized into groups and treated by administration via intraperitoneal injection of: G1: PBS, G2: 3 mg/kg Trastuzumab combined with 3 mg/kg Pertuzumab, G3: 3.6 mg/kg PRS-343, G4: 3 mg/kg Enhertu (DS8201), G5: 1.8 mg/kg HER2x4-1BB-1 combined with 1.8 mg/kg HER2x4-1BB-3, G6: 3.6 mg/kg HER2xHER2x4-1BB-1 (all groups were treated with an equivalent molar dose). The changes in tumor volume and body weight of mice in each group were monitored every 3-6 days/time for a duration of 2-3 weeks. The dosage and administration schedule were outlined in Table 10.

The results were shown in Figure 12. All mice treated with the HER2xHER2x4-1BB trispecific antibody exhibited complete tumor regression, demonstrating significantly superior efficacy compared to both the equimolar control antibody PRS-343 (TGI: 54.9%) and the combination of HER2 monoclonal antibodies (Trastuzumab + Pertuzumab, TGI: 63.8%). Furthermore, in this tumor model, the group treated with the combination of HER2x4-1BB bispecific antibodies (HER2x4-1BB-1 + HER2x4-1BB-3) and the group treated with Enhertu also achieved complete tumor regression.

**Table 10: Dosage regimen for the study on tumor inhibitory activity of the multi-specific antibodies**

| Group | Dosage | Number of dosages × dosage frequency |
|---|---|---|
| G1: Negative control (PBS) | N/A | Q3d × 2 |
| G2: Trastuzumab+Pertuzumab | 3+3 mg/kg | Q3d × 2 |
| G3: PRS-343 | 3.6mg/kg | Q3d × 2 |
| G4: Enhertu | 3 mg/kg | Q3d × 2 |
| G5: HER2x4-1BB-1+ HER2x4-1BB-3 | 1.8+1.8mg/kg | Q3d × 2 |
| G6: HER2x HER2x4-1BB-1 | 3.6 mg/kg | Q3d × 2 |

| | | |
|---|---|---|
| 1. Yarden, Y. and M.X. Sliwkowski, Untangling the ErbB signalling network. Nat Rev Mol Cell Biol, 2001. 2(2): p. 127-37. 2. Oh, D.Y. and Y.J. Bang, HER2-targeted therapies - a role beyond breast cancer. Nat Rev Clin Oncol, 2020. 17(1): p. 33-48. 3. Schaer, D.A., D. Hirschhorn-Cymerman, and J.D. Wolchok, Targeting tumor-necrosis factor receptor pathways for tumor immunotherapy. J Immunother Cancer, 2014. 2: p. 7. 4. Chen, S., et al., Combination of 4-1BB agonist and PD-1 antagonist promotes antitumor effector/memory CD8 T cells in a poorly immunogenic tumor model. Cancer Immunol Res, 2015. 3(2): p. 149-60. | | |

Although the specific embodiments of the present invention have been described in detail, those skilled in the art will understand that various modifications and substitutions can be made to those details based on all the teachings disclosed, and these changes are within the scope of the present invention. The full scope of the present invention is given by the appended claims and any equivalents thereof.

## Claims

1. A single-domain antibody or antigen-binding fragment thereof capable of specifically binding to 4-1BB, wherein the single-domain antibody comprises:
(a) a CDR1, which has a sequence as set forth in SEQ ID NO: 43, or a sequence having a substitution, deletion or addition of one or more amino acids (e.g., a substitution, deletion or addition of 1, 2 or 3 amino acids) as compared to the sequence as set forth in SEQ ID NO: 43;
(b) a CDR2, which has a sequence as set forth in SEQ ID NO: 44, or a sequence having a substitution, deletion or addition of one or more amino acids (e.g., a substitution, deletion or addition of 1, 2 or 3 amino acids) as compared to the sequence as set forth in SEQ ID NO: 44; and
(c) a CDR3, which has a sequence as set forth in SEQ ID NO: 45, or a sequence having a substitution, deletion or addition of one or more amino acids (e.g., a substitution, deletion or addition of 1, 2 or 3 amino acids) as compared to the sequence as set forth in SEQ ID NO: 45;
preferably, the substitution is a conservative substitution;
preferably, the single-domain antibody or antigen-binding fragment thereof comprises a CDR1 as set forth in SEQ ID NO: 43, a CDR2 as set forth in SEQ ID NO: 44, and a CDR3 as set forth in SEQ ID NO: 45.

2. The single-domain antibody or antigen-binding fragment thereof according to claim 1, wherein the single-domain antibody comprises an amino acid sequence selected from the following:
(i) a sequence as set forth in SEQ ID NO: 7;
(ii) a sequence having a substitution, deletion or addition of one or more amino acids (e.g., a substitution, deletion or addition of 1, 2, 3, 4 or 5 amino acids) as compared to the sequence as set forth in SEQ ID NO: 7; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 7;
preferably, the substitution is a conservative substitution.

3. A polypeptide construct capable of specifically binding to 4-1BB, which comprises the single-domain antibody or antigen-binding fragment thereof according to claim 1 or 2, and an immunoglobulin Fc domain;
preferably, the immunoglobulin Fc domain is linked directly or via a peptide linker to the N-terminal and/or C-terminal (e.g., C-terminal) of the single-domain antibody or antigen-binding fragment thereof; preferably, the peptide linker has a structure represented by (GₘXₙ)ₗG_{m'}, wherein m, m', n and l are each independently selected from 0, 1, 2, 3, 4, 5, 6, 7, 8, 9 and 10, and X is selected from A and S; preferably, m is selected from 1, 2, 3, 4 and 5, n is selected from 1 and 2, l is selected from 1, 2, 3, 4, 5, 6, 7, 8, 9 and 10, m' is selected from 0 and 1, and X is selected from A and S; preferably, the peptide linker has an amino acid sequence as set forth in SEQ ID NO: 17 or 18; preferably, the immunoglobulin Fc domain is the Fc domain of IgG (e.g., the Fc domain of IgG1, for example, comprising CH2 and CH3);
preferably, the immunoglobulin Fc domain comprises a sequence as set forth in SEQ ID NO: 19, or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto, or a sequence having a substitution, deletion or addition of one or more amino acids (e.g., a substitution, deletion or addition of 1, 2, 3, 4 or 5 amino acids) as compared thereto;
preferably, the immunoglobulin Fc domain comprises a sequence as set forth in SEQ ID NO: 19 or 20;
preferably, the polypeptide construct comprises or consists of an amino acid sequence as set forth in SEQ ID NO: 31 or 32.

4. A multi-specific antibody, which comprises the single-domain antibody or antigen binding fragment thereof according to claim 1 or 2, or the polypeptide construct according to claim 3;
preferably, the multi-specific antibody is capable of specifically binding to 4-1BB, and additionally specifically binding to one or more other targets;
preferably, the target is a tumor antigen;
preferably, the tumor antigen is one or more selected from the group consisting of: CD19, CD20, CD22, CD23, CD38, CD40, CD49, CD52, CD56, CD74, CD80, CD95, CD138, CS1/SLAMF7, KiR, Thy-1, Ly-6, Fas, APO-1, EGFR, HER2, CXCR4, HLA, GM1 and DRD.

5. A multi-specific antibody, which comprises a first antigen-binding domain specific for 4-1BB and a second antigen-binding domain specific for HER2, wherein,
the first antigen-binding domain comprises the antibody or antigen binding fragment thereof according to claim 1 or 2;
the second antigen-binding domain comprises at least one CDR of the heavy chain variable region of an anti-HER2 antibody, and/or at least one CDR of the light chain variable region of an anti-HER2 antibody;
preferably, the second antigen-binding domain comprises the heavy chain variable region of an anti-HER2 antibody, and/or the light chain variable region of an anti-HER2 antibody;
preferably, the anti-HER2 antibody is selected from the group consisting of trastuzumab, pertuzumab, and variants thereof;
the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived.

6. A multi-specific antibody, which comprises a first antigen-binding domain specific for 4-1BB and a second antigen-binding domain specific for HER2; wherein,
the first antigen-binding domain is VHH; the second antigen-binding domain is Fab, and the multi-specific antibody comprises:
(1) a peptide chain I-A, which comprises a light chain variable region and a light chain constant region (CL) of the second antigen-binding domain;
and,
(2) a peptide chain I-B, which comprises a heavy chain variable region and a heavy chain constant region of the second antigen-binding domain, and the first antigen-binding domain; preferably, the peptide chain I-B comprises, from the N-terminal to the C-terminal, the adjacent heavy chain variable region and heavy chain constant region of the second antigen-binding domain, and the first antigen-binding domain;
preferably, the CL of the peptide chain I-A is capable of forming a dimer with the heavy chain constant region CH1 domain of the peptide chain I-B;
preferably, the multi-specific antibody comprises two peptide chains I-A and two peptide chains I-B; preferably, the heavy chain constant regions of the two peptide chains I-B form a dimer;
preferably, the domains are linked directly or through a peptide linker; preferably, the peptide linker has a structure represented by (GₘXₙ)ₗG_{m'}, wherein m, m', n and l are each independently selected from 0, 1, 2, 3, 4, 5, 6, 7, 8, 9 and 10, and X is selected from A and S; preferably, m is selected from 1, 2, 3, 4 and 5, n is selected from 1 and 2, 1 is selected from 1, 2, 3, 4, 5, 6, 7, 8, 9 and 10, m' is selected from 0 and 1, and X is selected from A and S; preferably, the peptide linker has an amino acid sequence as set forth in SEQ ID NO: 17 or 18.

7. The multi-specific antibody according to claim 5 or 6, **characterized by** one or more of the following items:
(i) the first antigen-binding domain comprises or consists of an amino acid sequence as set forth in SEQ ID NO: 7;
(ii) the heavy chain variable region of the second antigen-binding domain comprises or consists of an amino acid sequence as set forth in SEQ ID NO: 1, 3 or 5;
(iii) the light chain variable region of the second antigen-binding domain comprises or consists of an amino acid sequence as set forth in SEQ ID NO: 2, 4 or 6;
preferably, the peptide chain I-A comprises or consists of an amino acid sequence as set forth in SEQ ID NO: 22, 24 or 26;
preferably, the peptide chain I-B comprises or consists of an amino acid sequence as set forth in SEQ ID NO: 21, 23 or 25.

8. The multi-specific antibody according to any one of claims 4 to 7, which further comprises a third antigen-binding domain specific for HER2;
preferably, the first antigen-binding domain is VHH; the second antigen-binding domain and the second antigen-binding domain are Fab, and the multi-specific antibody comprises:
(1) a peptide chain II-A, which comprises a light chain variable region and a light chain constant region (CL) of the second antigen-binding domain;
(2) a peptide chain II-B, which comprises a heavy chain variable region, a heavy chain constant region of the second antigen-binding domain, and the first antigen-binding domain; preferably, the peptide chain II-B comprises, from the N-terminal to the C-terminal, the adjacent heavy chain variable region and heavy chain constant region of the second antigen-binding domain, and the first antigen-binding domain;
(3) a peptide chain II-C, which comprises a light chain variable region and a chain constant region (CL) of the third antigen-binding domain;
and,
(4) a peptide chain II-D, which comprises a heavy chain variable region, a heavy chain constant region of the third antigen-binding domain, and the first antigen-binding domain; preferably, the peptide chain II-D comprises, from the N-terminal to the C-terminal, the adjacent heavy chain variable region and heavy chain constant region of the third antigen-binding domain, and the first antigen-binding domain;
preferably, the CL of the peptide chain II-A is capable of forming a dimer with the heavy chain constant region CH1 domain of the peptide chain II-B; preferably, the CL of the peptide chain II-C is capable of forming a dimer with the heavy chain constant region CH1 domain of the peptide chain II-D; preferably, the heavy chain constant region of the peptide chain II-B comprises an amino acid sequence as set forth in SEQ ID NO: 13, and the heavy chain constant region of the peptide chain II-D comprises an amino acid sequence as set forth in SEQ ID NO: 14; preferably, the heavy chain constant region of the peptide chain II-B comprises an amino acid sequence as set forth in SEQ ID NO: 37, and the heavy chain constant region of the peptide chain II-D comprises an amino acid sequence as set forth in SEQ ID NO: 38;
preferably, the multi-specific antibody comprises one peptide chain II-A, one peptide chain II-B, one peptide chain II-C, and one peptide chain II-D; preferably, the heavy chain constant regions of the peptide chains II-B and II-D form a dimer;
preferably, the domains are linked directly or through a peptide linker; preferably, the peptide linker has a structure represented by (GₘXₙ)ₗG_{m'}, wherein m, m', n and l are each independently selected from 0, 1, 2, 3, 4, 5, 6, 7, 8, 9 and 10, and X is selected from A and S; preferably, m is selected from 1, 2, 3, 4 and 5, n is selected from 1 and 2, 1 is selected from 1, 2, 3, 4, 5, 6, 7, 8, 9 and 10, m' is selected from 0 and 1, and X is selected from A and S; preferably, the peptide linker has an amino acid sequence as set forth in SEQ ID NO: 17 or 18.

9. The multi-specific antibody according to claim 8, **characterized by** one or more of the following items:
(i) the first antigen-binding domain comprises or consists of an amino acid sequence as set forth in SEQ ID NO: 7;
(ii) the heavy chain variable region of the second antigen-binding domain comprises or consists of an amino acid sequence as set forth in SEQ ID NO: 1, 3 or 5;
(iii) the light chain variable region of the second antigen-binding domain comprises or consists of an amino acid sequence as set forth in SEQ ID NO: 2, 4 or 6;
(iv) the heavy chain variable region of the third antigen-binding domain comprises or consists of an amino acid sequence as set forth in SEQ ID NO: 1, 3 or 5;
(v) the light chain variable region of the third antigen-binding domain comprises or consists of an amino acid sequence as set forth in SEQ ID NO: 2, 4 or 6;
preferably, the peptide chain II-A comprises or consists of an amino acid sequence as set forth in SEQ ID NO: 28 or 40;
preferably, the peptide chain II-B comprises or consists of an amino acid sequence as set forth in SEQ ID NO: 27 or 39;
preferably, the peptide chain II-C comprises or consists of an amino acid sequence as set forth in SEQ ID NO: 30 or 42;
preferably, the peptide chain II-D comprises or consists of an amino acid sequence as set forth in SEQ ID NO: 29 or 41.

10. A polynucleotide, which encodes the antibody according to any one of claims 1 to 9.

11. A vector, which comprises the polynucleotide according to claim 10.

12. A recombinant cell, which comprises the polynucleotide according to claim 10 or the vector according to claim 11.

13. A pharmaceutical composition, which comprises the antibody or antigen-binding fragment thereof according to any one of claims 1, 2, 4 to 9, the polypeptide construct according to claim 3, the polynucleotide according to claim 10, the vector according to claim 11 or the recombinant cell according to claim 12, and a pharmaceutically acceptable excipient.

14. Use of the antibody or antigen-binding fragment thereof according to any one of claims 1, 2, 4 to 9, the polypeptide construct according to claim 3, the polynucleotide according to claim 10, the vector according to claim 11, the recombinant cell according to claim 12 or the pharmaceutical composition according to claim 13, in the manufacture of a medicament for tumor treatment;
preferably, the tumor overexpresses HER2;
preferably, the tumor is selected from the group consisting of breast cancer, colon cancer, gastric cancer, lung cancer (e.g., lung squamous cell carcinoma, small cell lung cancer, non-small cell lung cancer, lung adenocarcinoma), peritoneal cancer, skin cancer, squamous cell carcinoma, skin or eyeball melanoma, rectal cancer, cancer near anus, esophageal cancer, small intestinal tumor, endocrine gland cancer, parathyroid cancer, adrenal cancer, soft tissue sarcoma, urethral cancer, chronic or acute leukemia, lymphocytic lymphoma, liver cancer, gastrointestinal cancer, pancreatic cancer, glioblastoma, cervical cancer, ovarian cancer, liver cancer, bladder cancer, hepatocellular adenoma, colorectal cancer, endometrial cancer or uterine cancer, salivary gland tumor, kidney cancer, cervical cancer, prostate cancer, vulvar cancer, thyroid cancer, head and neck cancer, brain cancer, biliary tract cancer and gallbladder cancer;
preferably, the tumor is a primary or metastatic tumor.

15. The antibody or antigen-binding fragment thereof according to any one of claims 1, 2, 4 to 9, the polypeptide construct according to claim 3, the polynucleotide according to claim 10, the vector according to claim 11, the recombinant cell according to claim 12 or the pharmaceutical composition according to claim 13, which is used for tumor treatment;
preferably, the tumor overexpresses HER2;
preferably, the tumor is selected from the group consisting of breast cancer, colon cancer, gastric cancer, lung cancer (e.g., lung squamous cell carcinoma, small cell lung cancer, non-small cell lung cancer, lung adenocarcinoma), peritoneal cancer, skin cancer, squamous cell carcinoma, skin or eyeball melanoma, rectal cancer, cancer near anus, esophageal cancer, small intestinal tumor, endocrine gland cancer, parathyroid cancer, adrenal cancer, soft tissue sarcoma, urethral cancer, chronic or acute leukemia, lymphocytic lymphoma, liver cancer, gastrointestinal cancer, pancreatic cancer, glioblastoma, cervical cancer, ovarian cancer, liver cancer, bladder cancer, hepatocellular adenoma, colorectal cancer, endometrial cancer or uterine cancer, salivary gland tumor, kidney cancer, cervical cancer, prostate cancer, vulvar cancer, thyroid cancer, head and neck cancer, brain cancer, biliary tract cancer and gallbladder cancer;
preferably, the tumor is a primary or metastatic tumor.

16. A method for tumor treatment, which comprises administering to a subject in need thereof a therapeutically effective amount of the antibody or antigen-binding fragment thereof according to any one of claims 1, 2, 4 to 9, the polypeptide construct according to claim 3, the polynucleotide according to claim 10, the vector according to claim 11, the recombinant cell according to claim 12, or the pharmaceutical composition according to claim 13;
preferably, the tumor overexpresses HER2;
preferably, the tumor is selected from the group consisting of breast cancer, colon cancer, gastric cancer, lung cancer (e.g., lung squamous cell carcinoma, small cell lung cancer, non-small cell lung cancer, lung adenocarcinoma), peritoneal cancer, skin cancer, squamous cell carcinoma, skin or eyeball melanoma, rectal cancer, cancer near anus, esophageal cancer, small intestinal tumor, endocrine gland cancer, parathyroid cancer, adrenal cancer, soft tissue sarcoma, urethral cancer, chronic or acute leukemia, lymphocytic lymphoma, liver cancer, gastrointestinal cancer, pancreatic cancer, glioblastoma, cervical cancer, ovarian cancer, liver cancer, bladder cancer, hepatocellular adenoma, colorectal cancer, endometrial cancer or uterine cancer, salivary gland tumor, kidney cancer, cervical cancer, prostate cancer, vulvar cancer, thyroid cancer, head and neck cancer, brain cancer, biliary tract cancer and gallbladder cancer;
preferably, the tumor is a primary or metastatic tumor.

17. A conjugate, which comprises the antibody or antigen-binding fragment thereof according to any one of claims 1, 2, 4 to 9, or the polypeptide construct according to claim 3, and a detectable label attached to the antibody or antigen-binding fragment thereof or the polypeptide construct;
preferably, the detectable label is selected from the group consisting of an enzyme (e.g., horseradish peroxidase or alkaline phosphatase), a chemiluminescent agent (e.g., an acridinium ester, luminol and a derivative thereof, or a ruthenium derivative), a fluorescent dye (e.g., fluorescein or fluorescent protein), a radionuclide or biotin.

18. A kit, which comprises the antibody or antigen-binding fragment thereof according to any one of claims 1, 2, 4 to 9, the polypeptide construct according to claim 3, or the conjugate according to claim 17;
preferably, the kit further comprises a second antibody capable of specifically recognizing the antigen specifically recognized by the antibody or antigen-binding fragment thereof or the polypeptide construct; optionally, the second antibody further comprises a detectable label, such as an enzyme (e.g., horseradish peroxidase or alkaline phosphatase), a chemiluminescent agent (e.g., an acridinium ester, luminol and a derivative thereof, or a ruthenium derivative), a fluorescent dye (e.g., fluorescein or fluorescent protein), a radionuclide or biotin.

19. A method for detecting the presence or level of 4-1BB in a sample, comprising using the antibody or antigen-binding fragment thereof according to any one of claims 1, 2, 4 to 9, the polypeptide construct according to claim 3, or the conjugate according to claim 17;
preferably, the method is an immunological assay, such as immunoblotting, enzyme immunoassay (e.g., ELISA), chemiluminescent immunoassay, fluorescent immunoassay or radioimmunoassay;
preferably, the method comprises using the antibody or antigen-binding fragment thereof or the polypeptide construct, and the method further comprises using a second antibody carrying a detectable label (e.g., an enzyme (e.g., horseradish peroxidase or alkaline phosphatase), a chemiluminescent agent (e.g., an acridinium ester, luminol and a derivative thereof, or a ruthenium derivative), a fluorescent dye (e.g., fluorescein or fluorescent protein), a radionuclide or biotin) to detect the binding of the antibody or antigen-binding fragment thereof or the polypeptide construct to an antigen;
preferably, the method comprises: (1) contacting the sample with the antibody or antigen-binding fragment thereof; (2) detecting the formation of an antigen-antibody immune complex or detecting the amount of the immune complex, wherein the formation of the immune complex indicates the presence of 4-1BB or a cell expressing 4-1BB.

20. Use of the antibody or antigen-binding fragment thereof according to any one of claims 1, 2, 4 to 9, the polypeptide construct according to claim 3, or the conjugate according to claim 17, in the manufacture of a detection reagent, wherein the detection reagent is used to detect the presence or level of 4-1BB in a sample;
preferably, the detection reagent detects the presence or level of 4-1BB in the sample by the method according to claim 19;
preferably, the sample is a cell sample (e.g., a tumor cell) from a subject (e.g., a mammal, preferably a human or a monkey).
